# EUROPEAN PATENT APPLICATION

(11) **EP 4 008 353 A1**
(43) Date of publication of application: **08.06.2022**
(21) Application number: 20846930.4
(22) Date of filing: 30.07.2020
(51) Int. Cl.: A61K 45/00, A61K 45/06, A61P 27/16, A61P 43/00, C07K 14/485, A61K 47/02, A61K 47/12, A61K 47/32, A61K 47/34, A61K 38/02, A61K 31/7088

(54) **PHARMACEUTICAL COMPOSITION FOR OTIC ADMINISTRATION**

(30) Priority: 31.07.2019 JP 2019140977
(71) Applicant: Astellas Pharma Inc., Chuo-ku Tokyo 103-8411 (JP)
(72) Inventor: YOSHIDA Takatsune, Tokyo 103-8411 (JP); OSADA Mari, Tokyo 103-8411 (JP); MATSUDA Takaya, Tokyo 103-8411 (JP); SHIMADA Ken, Tokyo 103-8411 (JP); KOJIMA Hiroyuki, Tokyo 103-8411 (JP); NAGAKURA Akira, Tokyo 103-8411 (JP)
(74) Representative: Hoffmann Eitle
(86) International application number: PCT/JP2020/029342
(87) International publication number: WO 2021/020535

(57) **Abstract**

Provided is a pharmaceutical composition that can be administered into the ear without any complicated operation and has a function of allowing a drug to be retained and slowly released in the ear. A pharmaceutical composition for otic administration, comprising one, or two or more drugs and a polymer, wherein when the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity is less than 1,650 mPa·s at 25°C, and is from 1,650 mPa·s to 100,000 mPa·s at 10 minutes after reaching 37°C.

SEQUENCE LISTING

<110>  Astellas Pharma Inc.

<120>  Pharmaceutical composition for otic administration

<130>  A20014-047

<150>  JP 2019-140977
<151>  2019-07-31

<160>  1

<170>  PatentIn version 3.5

<210>  1
<211>  86
<212>  PRT
<213>  Homo sapiens

<400>  1

Asp Leu Gln Glu Ala Asp Leu Asp Leu Leu Arg Val Thr Leu Ser Ser
1               5                   10                  15

Lys Pro Gln Ala Leu Ala Thr Pro Asn Lys Glu Glu His Gly Lys Arg
            20                  25                  30

Lys Lys Lys Gly Lys Gly Leu Gly Lys Lys Arg Asp Pro Cys Leu Arg
        35                  40                  45

Lys Tyr Lys Asp Phe Cys Ile His Gly Glu Cys Lys Tyr Val Lys Glu
    50                  55                  60

Leu Arg Ala Pro Ser Cys Ile Cys His Pro Gly Tyr His Gly Glu Arg
65                  70                  75                  80

Cys His Gly Leu Ser Leu
                85

SEQUENCE LISTING

<110>  Astellas Pharma Inc.

<120>  Pharmaceutical composition for otic administration

<130>  A20014-047

<150>  JP 2019-140977
<151>  2019-07-31

<160>  1

<170>  PatentIn version 3.5

<210>  1
<211>  86
<212>  PRT
<213>  Homo sapiens

<400>  1

Asp Leu Gln Glu Ala Asp Leu Asp Leu Leu Arg Val Thr Leu Ser Ser
1               5                   10                  15

Lys Pro Gln Ala Leu Ala Thr Pro Asn Lys Glu Glu His Gly Lys Arg
            20                  25                  30

Lys Lys Lys Gly Lys Gly Leu Gly Lys Lys Arg Asp Pro Cys Leu Arg
        35                  40                  45

Lys Tyr Lys Asp Phe Cys Ile His Gly Glu Cys Lys Tyr Val Lys Glu
    50                  55                  60

Leu Arg Ala Pro Ser Cys Ile Cys His Pro Gly Tyr His Gly Glu Arg
65                  70                  75                  80

Cys His Gly Leu Ser Leu
                85

## Description

### TECHNICAL FIELD

The present invention relates to a pharmaceutical composition that can be administered into the ear without any complicated operation and has a function of allowing a drug to be retained and slowly released in the ear.

### BACKGROUND ART

Regarding ear diseases, the number of drug therapy methods is still small, and frequently, the treatment depends on surgical operations. Furthermore, even if a drug effective for the treatment is discovered, when the drug is systemically delivered by oral administration or intravenous administration, there is a risk of a decline in efficacy, adverse side effects, and the like. Therefore, medicines or pharmaceutical compositions for locally administering a drug into a diseased site of the ear or the vicinity thereof have been developed. For example, there are ear drops, ointments, and the like, which contain antibiotic substances or steroid drugs as anti-inflammatory agents for the mucous membrane and the like of the ear. When a medicine is administered in a cold state into the ear, the drug may cause dizziness. Therefore, when an ear drop is administered, the user is required to grip the container of the medicine with the palm of the hand and to warm the medicine to a temperature close to the body temperature. Furthermore, at the time of administering, the user is required to lie on one side, perform ear dripping for the indicated number of times, and maintain the posture as it is for approximately 5 to 10 minutes. Since the ear has a function of clearing foreign matter such as a liquid from the external auditory canal and the Eustachian tube of the middle ear, and the clearance is promoted by swallowing, it is necessary for the user to rest in bed and endure swallowing until the medicine penetrates into the diseased part. Furthermore, in the case of delivering a drug into the inner ear, since there exists the blood-inner ear barrier, sustained release properties by which a medicine at a high concentration is caused to act sustainedly at a local site, are required.

In Patent literature 1 (WO 2001/068079), it is reported to drip sodium hyaluronate, which is a viscoelastic agent, during a surgical treatment or at the time of completion thereof, and to retain the medicine inside the ear. Hyaluronic acid is a kind of mucopolysaccharide and is an essential constituent component of the extracellular matrix in the living body, and hyaluronic acid has been generally used as a biomaterial.

In Patent literature 2 (WO 2014/186075), a therapeutic method of producing a sustained release gel by incorporating an agent into a crosslinked copolymer hydrogel of chitosan and polylactide, and causing the agent to be retained on a tympanic membrane perforation using an injector syringe, is reported. Chitosan and polylactide, which is a hydrolysable crosslinking agent, have characteristics of undergoing chemical crosslinking as a result of mixing immediately before administration or during administration, and changing into a crosslinked copolymer hydrogel within several minutes.

In Patent literature 3 (WO 2011/049958), a sustained release gel preparation utilizing a thermo-reversible gel is reported as a technology that does not require preliminary mixing or a special administration device. It is reported that when a solution obtained by dissolving a polyoxyethylene-polyoxypropylene triblock copolymer, which is a constituent component of a thermo-reversible gel, at a concentration of approximately 14% by weight to approximately 21% by weight is administered onto the round window membrane or the vicinity thereof by means of an injection needle, the solution is warmed in the ear (in Non-Patent literature 1: James M. Chamberlain et al., ANNALS OF ENERGENCY MEDICINE, 1995, 25(1), p. 15-20, it is reported that the temperature inside the external auditory canal is approximately 37°C to approximately 38°C) to gelate, and the gel is retained on the round window membrane or in the vicinity thereof. Since the above-described sustained release gel preparation gelates at a relatively low temperature, strict temperature management is needed for the preparation before administration.

A plurality of pharmaceutical compositions having a function by which a drug is retained and slowly released in the ear has been reported. However, in order to provide a pharmaceutical composition that can be administered into the ear without any complicated operation, such as mixing of two liquids immediately before administration or during administration, or strict temperature management, and has a function of allowing a drug to be retained and slowly released in the ear, there is room for further investigation.

### CITATION LIST

### PATENT LITERATURE

[Patent literature 1] WO 2001/068079
[Patent literature 2] WO 2014/186075
[Patent literature 3] WO 2011/049958

### NON-PATENT LITERATURE

[Non-patent literature 1] James M Chamberlain et al., ANNALS OF EMERGENCY MEDICINE, 1995, 25(1), p.15-20

### SUMMARY OF INVENTION

### TECHNICAL PROBLEM

An object of the present invention is to provide a pharmaceutical composition that can be administered into the ear without any complicated operation and has a function of allowing a drug to be retained and slowly released in the ear.

### SOLUTION TO PROBLEM

The inventors of the present invention conducted a thorough investigation on a pharmaceutical composition that can be administered at room temperature and has a function of retaining and slowly releasing a drug in the ear, and as a result, the inventors found a pharmaceutical composition comprising one, or two or more drugs and a polymer, and having particular complex viscosity characteristics. In addition, the present inventors found that a pharmaceutical composition that can be administered into the ear without any complicated operation and has a function of allowing a drug to be retained and slowly released in the ear can be provided by using a polyvinyl caprolactam-polyvinyl acetate-polyethylene glycol graft copolymer as the polymer, and completed the present invention.

The present invention relates to:
[1] a pharmaceutical composition for otic administration, comprising one, or two or more drugs and a polymer, wherein when a complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity is less than 1,650 mPa·s at 25°C, and is from 1,650 mPa·s to 100,000 mPa·s at 10 minutes after reaching 37°C;
[2] the pharmaceutical composition of [1], wherein a sol-gel transition point is 30°C to 38°C;
[3] the pharmaceutical composition of [1] to [2], wherein the polymer is a polyvinyl caprolactam-polyvinyl acetate-polyethylene glycol graft copolymer;
[4] the pharmaceutical composition of any one of [1] to [3], further comprising one, or two or more agents for adjusting complex viscosity;
[5] The pharmaceutical composition of [4], wherein the agent for adjusting complex viscosity is one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof;
[6] the pharmaceutical composition of [4] or [5], wherein the agent for adjusting complex viscosity is one, or two or more compounds selected from the group consisting of trisodium citrate, tripotassium citrate, sodium sulfate, and hydrates thereof;
[7] the pharmaceutical composition of any one of [1] to [6], further comprising a solvent;
[8] the pharmaceutical composition of any one of [4] to [7], wherein a concentration of the polyvinyl caprolactam-polyvinyl acetate-polyethylene glycol graft copolymer is 10%(w/w) to 24%(w/w) with respect to the pharmaceutical composition;
[9] the pharmaceutical composition of any one of [4] to [8], wherein a concentration of the agent for adjusting complex viscosity is 0.1%(w/w) to 5.0%(w/w) with respect to the pharmaceutical composition;
[10] the pharmaceutical composition of any one of [1] to [9], wherein the drug is slowly released, when a thin, semipermeable membrane-attached insert is inserted into a multi-well plate containing 2 mL of a phosphate buffered saline at 37°C ± 2°C so that the phosphate buffered saline is divided into an upper layer and a lower layer, 0.5 mL of a pharmaceutical composition is added to the insert of upper layer, and the drug is dissolved by shaking the multi-well plate at a rate at which the water surface of the phosphate buffered saline shakes;
[11] the pharmaceutical composition of any one of [1] to [10], wherein the drug is one, or two or more compounds selected from the group consisting of a small molecule compound, a nucleic acid, and a protein;
[12] the pharmaceutical composition of any one of [1] to [11], wherein the drug is a drug that provides a biological activity of a heparin-binding epidermal growth factor;
[13] the pharmaceutical composition of [12], wherein the drug that provides a biological activity of a heparin-binding epidermal growth factor comprises a protein consisting of the amino acid sequence of SEQ ID NO: 1;
[14] a pharmaceutical composition for otic administration, comprising one, or two or more drugs, and a polyvinyl caprolactam-polyvinyl acetate-polyethylene glycol graft copolymer;
[15] the pharmaceutical composition of [14], further comprising one, or two or more agents for adjusting complex viscosity;
[16] the pharmaceutical composition of [15], wherein the agent for adjusting complex viscosity is one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof;
[17] the pharmaceutical composition of [15] or [16], wherein the agent for adjusting complex viscosity is one, or two or more compounds selected from the group consisting of trisodium citrate, tripotassium citrate, sodium sulfate, and hydrates thereof;
[18] the pharmaceutical composition of any one of [14] to [17], further comprising a solvent;
[19] the pharmaceutical composition of any one of [15] to [18], wherein a concentration of the polyvinyl caprolactam-polyvinyl acetate-polyethylene glycol graft copolymer is 10%(w/w) to 24%(w/w) with respect to the pharmaceutical composition;
[20] the pharmaceutical composition of any one of [15] to [19], wherein a concentration of the agent for adjusting complex viscosity is 0.1%(w/w) to 5.0%(w/w) with respect to the pharmaceutical composition;
[21] the pharmaceutical composition of any one of [14] to [20], wherein the drug is one, or two or more compounds selected from the group consisting of a small molecule compound, a nucleic acid, and a protein;
[22] the pharmaceutical composition of any one of [14] to [21], wherein the drug is a drug that provides a biological activity of a heparin-binding epidermal growth factor;
[23] the pharmaceutical composition of [22], wherein the drug that provides a biological activity of a heparin-binding epidermal growth factor comprises a protein consisting of the amino acid sequence of SEQ ID NO: 1;
[24] a pharmaceutical composition for otic administration, comprising a drug that provides a biological activity of a heparin-binding epidermal growth factor, a polyvinyl caprolactam-polyvinyl acetate-polyethylene glycol graft copolymer, and trisodium citrate or a hydrate thereof;
[25] use of a polyvinyl caprolactam-polyvinyl acetate-polyethylene glycol graft copolymer, as a base, for the manufacture of a pharmaceutical composition for otic administration comprising one, or two or more drugs; and
[26] use of one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof, as an agent for adjusting complex viscosity, for the manufacture of a pharmaceutical composition for otic administration comprising one, or two or more drugs, and a polyvinyl caprolactam-polyvinyl acetate-polyethylene glycol graft copolymer.

### ADVANTAGEOUS EFFECTS OF INVENTION

According to the present invention, there can be provided a pharmaceutical composition for otic administration comprising one, or two or more drugs and a polymer, particularly, a polyvinyl caprolactam-polyvinyl acetate-polyethylene glycol graft copolymer, the pharmaceutical composition being administrable into the ear without any complicated operation and having a function of allowing a drug to be retained and slowly released in the ear, wherein when the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity is less than 1,650 mPa·s at 25°C, and is from 1,650 mPa·s to 100,000 mPa·s at 10 minutes after reaching 37°C.

Further, according to the present invention, there can be provided a pharmaceutical composition for otic administration, the pharmaceutical composition comprising one, or two or more drugs and a polyvinyl caprolactam-polyvinyl acetate-polyethylene glycol graft copolymer, being administrable into the ear without any complicated operation, and having a function of allowing the drug to be retained and slowly released in the ear.

Furthermore, according to the present invention, there can be provided a pharmaceutical composition for otic administration that is excellent for the treatment of chronic tympanic membrane perforation, the pharmaceutical composition comprising a drug that provides a biological activity of a heparin-binding epidermal growth factor, a polyvinyl caprolactam-polyvinyl acetate-polyethylene glycol graft copolymer, and trisodium citrate or a hydrate thereof, being administrable into the ear without any complicated operation, and having a function of retaining and slowly releasing the drug that provides a biological activity of a heparin-binding epidermal growth factor in the ear.

### BRIEF DESCRIPTION OF THE DRAWINGS

Fig. 1 is a graph showing time courses of the complex viscosities of some base solutions in the measurement of the complex viscosities of base solutions carried out in Experimental Example 3-1.
Fig. 2 is a graph showing the relationship between the sol-gel transition points carried out in Experimental Example 5-3 and the complex viscosities at 10 minutes after reaching 37°C carried out in Experimental Example 5-2.
Fig. 3 is a graph showing dissolution profiles obtained in a dissolution test for acetaminophen carried out in Experimental Example 7-4.
Fig. 4 is a graph showing the evaluation results for the retention properties in the ear obtained by a test using a fluorescent dye carried out in Experimental Example 8.
Fig. 5 is a graph showing the evaluation results for the retention properties in the ear obtained by PET imaging carried out in Experimental Example 9.

### DESCRIPTION OF EMBODIMENTS

The present invention relates to a pharmaceutical composition for otic administration comprising one, or two or more drugs and a polymer, wherein when the complex viscosity of the pharmaceutical composition is measured using a rheometer under the conditions of a shear strain of 5% and an angular frequency of 50 rad/sec. while increasing the temperature from 25°C to 37°C at a heating rate of 1°C/10 sec., the complex viscosity is less than 1,650 mPa·s at 25°C, and is from 1,650 mPa·s to 100,000 mPa·s at 10 minutes after reaching 37°C. Further, the present invention relates to a pharmaceutical composition for otic administration comprising one, or two or more drugs and a polyvinyl caprolactam-polyvinyl acetate-polyethylene glycol graft copolymer. Furthermore, the present invention relates to a pharmaceutical composition for otic administration comprising a heparin-binding epidermal growth factor, a polyvinyl caprolactam-polyvinyl acetate-polyethylene glycol graft copolymer, and trisodium citrate or a hydrate thereof.

The ear is distinguished into the outer ear, the middle ear, and the inner ear. The outer ear is composed of the pinna, the external auditory canal, and outer membrane of the tympanic membrane. The middle ear is composed of inner membrane of the tympanic membrane, auditory ossicles (malleus, incus, and stapes), tympanic cavity, muscles of auditory ossicles, Eustachian tube, mastoid antrum, and mastoid air cells. The inner ear is composed of the oval window membrane, vestibule, semicircular canal, utricle, saccule, round window membrane, cochlear, and the like.

The term "otic administration" as used herein typically means administering a pharmaceutical composition into the external auditory canal, in the vicinity of outer membrane of the tympanic membrane, on the tympanic membrane, in the vicinity of inner membrane of the tympanic membrane, into the tympanic cavity, on the round window membrane, or in the vicinity of the round window, but it is not limited thereto. Preferably it means administering a pharmaceutical composition in the vicinity of outer membrane of the tympanic membrane, on the tympanic membrane, in the vicinity of inner membrane of the tympanic membrane, or into the tympanic cavity. The term "pharmaceutical composition "for otic administration"" as used herein typically means a pharmaceutical composition to be administered into the external auditory canal, in the vicinity of outer membrane of the tympanic membrane, on the tympanic membrane, in the vicinity of inner membrane of the tympanic membrane, into the tympanic cavity, on the round window membrane, or in the vicinity of the round window, but the term is not limited thereto. Preferably it means a pharmaceutical composition to be administered in the vicinity of outer membrane of the tympanic membrane, on the tympanic membrane, in the vicinity of inner membrane of the tympanic membrane, or into the tympanic cavity.

The pharmaceutical composition of the present invention may be in the form of a solid, a powder, a solution, a suspension, a paste, a lotion, or the like, but the form is not limited thereto. The form is preferably a solution, a suspension, a paste, or a lotion, and more preferably a solution or a suspension. In connection with this, the terms "sol state" and "gel state" as used herein can be included in the term "solution". The term "sol state" means a colloidal solution in which fine particles are dispersed in a liquid and has fluidity. The term "gel state" means that the viscosity in the sol state increases and the fluidity disappears to become solid.

The "polymer" used in the pharmaceutical composition of the present invention is a pharmaceutical additive for producing viscosity in the pharmaceutical composition, and typically there may comprise an amphiphilic polymer with temperature responsiveness (also referred to as heat sensitivity, thermoreversibility, or thermosetting), but the polymer is not limited thereto. As the amphiphilic polymer, for example, there may typically comprise an A-B-A type or B-A-B type block copolymer in which a hydrophilic block (A), such as polyvinyl caprolactam, polyethylene glycol, polypropylene oxide, or the like, and a hydrophobic block (B), such as polyvinyl acetic acid, ethylene oxide, or the like, are covalently linked, but it is not limited thereto. The A-B-A type block copolymer is preferable, and for example, there may typically comprise polyvinyl caprolactam-polyvinyl acetate-polyethylene glycol graft copolymer, PEG-PLGA-PEG triblock copolymer, or the like, but it is not limited thereto. The amphiphilic polymer is preferably polyvinyl caprolactam-polyvinyl acetate-polyethylene glycol graft copolymer.

The "polyvinyl caprolactam-polyvinyl acetate-polyethylene glycol graft copolymer" used in the pharmaceutical composition of the present invention is also referred to as polyethylene caprolactam-polyvinyl acetate-polyethylene glycol graft copolymer, polyvinyl caprolactam-polyvinyl acetate-polyethylene glycol graft copolymer, or PVCap-PVAc-PEG, and is amphiphilic represented by the structural formula (1) and has an average molecular weight (weight average molecular weight; Mw) of 90,000-140,000. As a commercial product, for example, it may be referred to as SOLUPLUS (registered trademark), but it is not limited thereto. 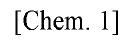 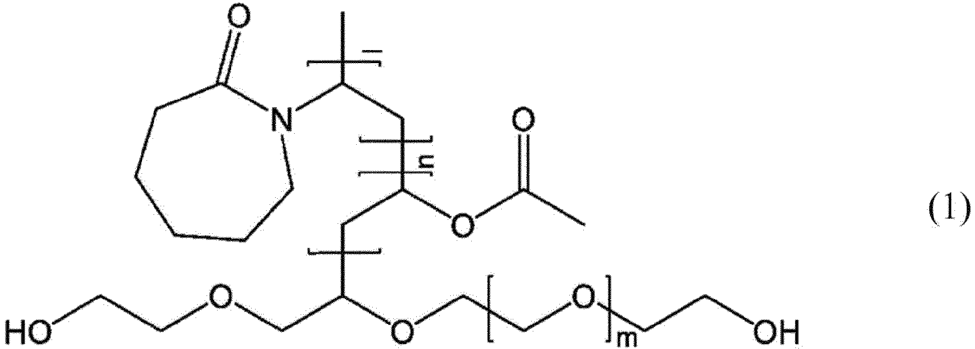 [l, m, and n are independently within the range of approximately 10 to 30,000.]

As a "solvent" used in the pharmaceutical composition of the present invention, or a solvent for dissolving or dispersing the pharmaceutical composition of the present invention, typically there may comprise purified water, water for injection, other pharmaceutically acceptable water, a mixture of water and a water-miscible solvent such as alkanol having 1 to 7 carbon atoms, a dextrose aqueous solution, or the like, but the solvent is not limited thereto. The solvent is preferably purified water, water for injection, or other pharmaceutically acceptable water.

The polymer used in the present invention can be added within the range in which the desired effects of the present invention are achieved. The concentration of the polymer, particularly polyvinyl caprolactam-polyvinyl acetate-polyethylene glycol graft copolymer, can be measured using a high performance liquid chromatography (HPLC) or an absorption spectrometer, when the polymer is contained in a solution. The lower limit of the concentration of the polymer, particularly polyvinyl caprolactam-polyvinyl acetate-polyethylene glycol graft copolymer, is typically 19%(w/w), preferably 20%(w/w), more preferably 25%(w/w), and even more preferably 27%(w/w), with respect to the pharmaceutical composition. The upper limit of the concentration of the polymer, particularly polyvinyl caprolactam-polyvinyl acetate-polyethylene glycol graft copolymer, is 29%(w/w).

When the pharmaceutical composition further contains the agent for adjusting complex viscosity, the concentration of the polymer, particularly polyvinyl caprolactam-polyvinyl acetate-polyethylene glycol graft copolymer, is typically 10%(w/w) to 24%(w/w), preferably 11%(w/w) to 23%(w/w), more preferably 13%(w/w) to 21%(w/w), and most preferably 15%(w/w) to 20%(w/w), with respect to the pharmaceutical composition. The upper limits and the lower limits can be arbitrarily combined if desired.

The term "complex viscosity" as used herein means a viscosity (SI unit is Pa·s) obtained by dynamic measurement using a rheometer (viscoelasticity measuring device). A method of measuring complex viscosity may be, for example, a method in which the measurement is carried out using a rheometer (MCR302, manufactured by Anton Paar) equipped with a plate (MEASURING CONE CP25-2), a hood (H-PTD200), and a gap of 0.3 mm, under the conditions of a sample volume of 0.3 mL, a shear strain of 5%, an angular frequency of 50 rad/sec., a normal force of 0 N, and a torque reliability range of 1 µN·m or more, but the method is not limited thereto. When the pharmaceutical composition does not have fluidity, such as in the case of a solid, a powder, or the like, the complex viscosity should be measured after being dissolved or dispersed in purified water, water for injection, or other pharmaceutically acceptable water.

It is preferable that the pharmaceutical composition of the present invention can be administered into the ear without any complicated operation. That is to say, it is preferable that the complex viscosity of the pharmaceutical composition is low enough to have fluidity, typically at ordinary temperature (15°C to 25°C), and preferably at room temperature (1 to 30°C). For example, when the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity at 25°C is typically less than 1,650 mPa·s, preferably less than 1,600 mPa·s, more preferably less than 1,500 mPa·s, more preferably less than 1,400 mPa·s, even more preferably less than 1,200 mPa·s, and most preferably less than 1,000 mPa·s. When the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity at 30°C is typically less than 1,650 mPa·s, preferably less than 1,600 mPa·s, more preferably less than 1,500 mPa·s, more preferably less than 1,400 mPa·s, even more preferably less than 1,200 mPa·s, and most preferably less than 1,000 mPa·s. The lower limit of the complex viscosity is 0 mPa·s.

It is preferable that the pharmaceutical composition of the present invention has the feature that, immediately after administration into the ear, the property changes to that showing retention in the ear. That is to say, it is preferable that, after administration into the ear, which is approximately 37°C to 38°C, the complex viscosity of the pharmaceutical composition rapidly becomes so high that it does not have fluidity. For example, when the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity at 1 minute, 5 minutes, or 10 minutes after reaching 37°C is typically 1,650 mPa·s or more, preferably 1,800 mPa·s or more, more preferably 2,000 mPa·s or more, even more preferably 2,500 mPa·s or more, and most preferably 3,000 mPa·s or more. If the complex viscosity is too high, the retained substance after administration may be released from the wall surface or the like in the ear and may be cleared, the complex viscosity at 37°C is typically 100,000 mPa·s or less, preferably 90,000 mPa·s or less, more preferably 80,000 mPa·s or less, even more preferably 70,000 mPa·s or less, and most preferably 60,000 mPa·s or less. The upper limits and the lower limits can be arbitrarily combined if desired.

As described above, it is preferable that the pharmaceutical composition that can be administered into the ear without any complicated operation and has a function of allowing the drug to be retained in the ear after the administration. That is to say, it is prefeferable that the complex viscosity of the pharmaceutical composition is low enough to have fluidity at ordinary temperature and/or room temperature, and is high enough not to have fluidity in the ear. For example, when the complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity is typically less than 1,650 mPa·s at 25°C and 1,650 mPa·s to 100,000 mPa·s at 10 minutes after reaching 37°C, preferably less than 1,600 mPa·s at 25°C and 1,800 mPa·s to 100,000 mPa·s at 10 minutes after reaching 37°C, more preferably less than 1,200 mPa·s at 25°C and 2,000 mPa·s to 80,000 mPa·s at 10 minutes after reaching 37°C, and even more preferably less than 1,000 mPa·s at 25°C and 3,000 mPa·s to 80,000 mPa·s at 10 minutes after reaching 37°C.

The "sol-gel transition point" as used herein means the temperature at the time when a pharmaceutical composition is converted from a sol state to a gel state. A method for evaluating the sol-gel transition point may be, for example, a method using a rheometer or the like, but it is not limited thereto. In the present specification, when the complex viscosity of the pharmaceutical composition is measured using, for example, a rheometer (MCR302) manufactured by Anton Paar, the storage modulus (G'), which is a solid element, and the loss modulus (G"), which is a liquid element, are simultaneously digitized, and when G' < G" is designated as a sol state, while G' > G" is designated as a gel state, the temperature at the time point when G' = G" is achieved can be designated as the sol-gel transition point. Incidentally, in a case in which the pharmaceutical composition does not have fluidity, such as in the case of a solid, a powder, or the like, the pharmaceutical composition should be dissolved or dispersed in purified water, water for injection, or other pharmaceutically acceptable water, and then the measurement should be carried out. A preferred range of the sol-gel transition point for the pharmaceutical composition according to the present invention is typically 30°C to 38°C, preferably 31°C to 37°C, more preferably 32°C to 36°C, and even more preferably 32°C to 35°C. The upper limits and the lower limits can be arbitrarily combined if desired.

The term "gelation" or "gel formation" as used herein means that a pharmaceutical composition or base having fluidity comes to lose fluidity, and the term "gel-forming ability" means a property of gelating. When a pharmaceutical composition has fluidity, since there is a risk that the retention rate of the pharmaceutical composition may be decreased, it is preferable that the pharmaceutical composition or the base rapidly forms a gel after otic administration. A method of evaluating the presence or absence of gel formation or the gel-forming ability may be a method of dropping 0.3 mL of a pharmaceutical composition or a base onto a plastic Petri dish or into a test tube, covering the Petri dish or the test tube with a lid, subsequently placing the Petri dish or the test tube on the water surface of a water bath set to 25°C or 37°C or on a heat block that can regulate the temperature of samples to 25°C or 37°C, taking out the plastic Petri dish or the like after 1 minute, after 5 minutes, after 10 minutes, after 15 minutes, or after 30 minutes from the beginning of placement, immediately inverting the Petri dish or the like, and checking the behavior of the pharmaceutical composition or the base by visual inspection for 10 seconds from the inversion (hereinafter also referred to as a gel-forming ability test), but the method is not limited thereto. It is preferable that a sample that has been placed for 1 minute, for 5 minutes, for 10 minutes, for 15 minutes, or for 30 minutes on the water surface of a water bath set to 25°C in the gel-forming ability test, typically drops or flows horizontally in a time period of less than 10 seconds from inversion, and preferably drops or flows horizontally in a time period of less than 5 seconds from inversion. Furthermore, it is preferable that, for a sample that has been placed for 1 minute or for 10 minutes on the water surface of a water bath set to 37°C in the gel-forming ability test, typically the sample dropping or horizontally-flowing cannot be observed in a time period of less than 5 seconds from inversion, preferably the sample dropping or horizontally-flowing cannot be observed in a time period of less than 10 seconds from inversion, and more preferably neither the sample dropping nor the sample horizontally-flowing can be observed for 10 seconds from inversion.

The term "base" as used herein means, among the constituent components of the pharmaceutical composition, a pharmaceutical additive having mainly a function of imparting complex viscosity, or having a function of allowing a drug to be retained and slowly released in the ear. More particularly, the base as used herein means typically the polymer, preferably the polymer and the agent for adjusting complex viscosity, and more preferably remaining pharmaceutical additives obtained by removing the drug from the pharmaceutical composition.

The term "agent for adjusting complex viscosity" means a pharmaceutical additive that increases or decreases the complex viscosity of the pharmaceutical composition. The agent for adjusting complex viscosity used in the present invention is not particularly limited, as long as it is pharmaceutically acceptable, and does not affect the sustained release properties of one, or two or more drugs contained in the pharmaceutical composition of the present invention. The agent for adjusting complex viscosity is typically trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, or hydrates thereof, or combinations thereof, preferably trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, or hydrates thereof, or combinations thereof, more preferably trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, or hydrates thereof, or combinations thereof, even more preferably trisodium citrate, tripotassium citrate, sodium sulfate, or hydrates thereof, or combinations thereof, and even more preferably trisodium citrate or hydrates thereof. Another embodiment of the agent for adjusting complex viscosity may be an organic solvent such as dichloromethane or the like, but it is not limited thereto.

The agent for adjusting complex viscosity can be added alone, or in combination of two or more within the range in which the desired effects of the present invention are achieved. The concentration of the agent for adjusting complex viscosity in the pharmaceutical composition can be measured using HPLC, an absorption spectrometer, or the like, when the pharmaceutical composition is in a solution state, but it is not limited thereto. The concentration of the agent for adjusting complex viscosity is, for example, typically 0.1%(w/v) to 5.0%(w/v), preferably 0.8%(w/v) to 3.1%(w/v), more preferably 1.0%(w/v) to 3.1%(w/v), more preferably 1.0%(w/v) to 2.5%(w/v), even more preferably 1.4%(w/v) to 2.4%(w/v), and most preferably 1.5%(w/v) to 2.2%(w/v). The upper limits and the lower limits can be arbitrarily combined if desired.

It is preferable that the "osmotic pressure" of the pharmaceutical composition of the present invention is within the range that is not irritating to the ear. Since the osmotic pressure of perilymph of the human ear is approximately 280 mOsm to approximately 300 mOsm, and high osmotic pressure is accompanied by risk of side effects, it is preferable that the osmotic pressure of the pharmaceutical composition is lower than that of the perilymph. When the osmotic pressure of the pharmaceutical composition of the present invention is measured, for example, using an automatic osmometer (OM-6050, manufactured by ARKRAY) or the like, it is typically 50 mOsm to 300 mOsm, preferably 90 mOsm to 300 mOsm, more preferably 180 mOsm to 300 mOsm, even more preferably 200 mOsm to 300 mOsm, and most preferably 220 mOsm to approximately 290 mOsm. The upper limits and the lower limits can be arbitrarily combined if desired.

It is preferable that the "pH" of the pharmaceutical composition of the present invention is not irritating to the ear, or is within the range that does not affect the stability of the pharmaceutical composition. The pH of the pharmaceutical composition means a pH in a solution state, when the form of the pharmaceutical composition is a solution state, and means a pH in a solution state obtained by dissolving the pharmaceutical composition in a solvent (for example, purified water, water for injection, or other pharmaceutically acceptable water), when the pharmaceutical composition is in a non-solution state. The pH of the pharmaceutical composition of the present invention is typically 3.0 to 10.5, preferably 4.5 to 9.0, more preferably 5.5 to 8.5, even more preferably 6.0 to 8.0, and most preferably 6.5 to 8.0. The upper limits and the lower limits can be arbitrarily combined if desired.

The recitation "a drug is slowly released" or "sustained release (properties)" means that, after administration of a pharmaceutical composition, a drug continues to dissolve for a certain period of time, or the properties that continue to dissolve. The dissolution rate is not particularly limited, but it is preferable that the rate of sustained drug release is slow, because the purpose of sustained drug release of the drug is to reduce the number of administrations, and/or, to maintain the drug effect and to reduce side effects by maintaining a constant drug concentration at a target site.

A method of measuring the sustained release properties of a drug, in an *in vitro* situation may be, for example, a method of inserting a thin, semipermeable membrane-attached insert into a multi-well plate containing 2 mL of a phosphate buffered saline (hereinafter also referred to as PBS) that has been warmed to 37°C ± 2°C, so that the PBS is divided into an upper layer and a lower layer, adding 0.5 mL of a pharmaceutical composition containing one, or two or more drugs on the insert of upper layer, shaking the multi-well plate at a rate at which the water surface of the PBS shakes (for example, a rate of 100 rpm in CHROMATO Chamber M-600FN manufactured by TAITEC), and thereby dissolving the drug (hereinafter also referred to as a dissolution test). The method has been made with reference to a method described in Dose-dependent sustained release of dexamethasone in inner ear cochlear fluids using a novel local delivery Approach (Xiaobo Wang, 2009 Audiol Neurotol 14:393-401), but the method of measuring the sustained release properties of a drug is not limited to the method. If the dissolution of a drug depends on pH and the dissolution properties cannot be properly evaluated, instead of PBS, a preferred test solution for each drug (for example, a solvent capable of completely (i.e., 100%) dissolving a certain amount of drug, or the like) may be used, for example, physiological saline or the like, but is not limited thereto.

In order to evaluate the sustained release properties of the pharmaceutical composition of the present invention by the dissolution test, the evaluation can be carried out, for example, by quantitatively determining the drug concentrations of the lower layer of the insert at not less than three points of time, for example, at 1, 6, and 24 hours after the beginning of the test, at 1, 4, and 8 hours after the beginning of the test, or at 1, 24, and 48 hours after the beginning of the test, and evaluating the time course of the drug dissolution rate. The method of quantitatively determining a drug is not particularly limited, and the most suitable analytical method for quantitative determination may be used. For example, there may comprise an HPLC, a fluorescence spectrometer, or the like, but it is not limited thereto. The recitation "a drug is slowly released" means that, in the above dissolution test, for example, the drug dissolution rate at 1 hour after beginning of the dissolution test is typically 40% or less, preferably 30% or less, more preferably 20% or less, even more preferably 15% or less, and most preferably 12% or less. Further, for example, the drug dissolution rate at 6 hours after beginning of the dissolution test is typically 70% or less, preferably 60% or less, more preferably 50% or less, even more preferably 40% or less, and most preferably 35% or less. Furthermore, for example, the drug dissolution rate at 24 hours after beginning of the dissolution test is typically 85% or less, preferably 75% or less, even more preferably 70% or less, even more preferably 60% or less, and most preferably 55% or less. In connection with this, the test solution used in the dissolution test is not limited to a phosphate buffer, and a preferred test solution for each drug (for example, a solvent capable of completely (i.e., 100%) dissolving a certain amount of drug, or the like) may be used.

The term "retention" or "retention properties" as used herein means that a pharmaceutical composition administered into the ear avoids the clearance of the outer ear and the middle ear, and remains at the administration site, or the properties that remain at the administration site. A method of evaluating the retention properties is not particularly limited. For example, there may comprise PET (positron emission tomography) imaging, SPECT (single photon emission computed tomography) imaging, a test using a fluorescent dye, MRI (magnetic resonance imaging), or the like, but the method is not limited thereto. The PET imaging or the SPECT imaging as used herein means a test in which a pharmaceutical composition containing a drug labeled with a radioisotope is administered to an administration site in the ear of a human or a model animal such as a monkey or rodents, and the residual amount of radioisotope in the ear after a certain period of time is quantitatively determined. The residual rate can be calculated from the administered amount and the residual amount. The test using a fluorescent dye as used herein means a test in which, for example, the pharmaceutical composition containing a drug in which a fluorescent dye is encapsulated or labeled is administered to an administration site in the ear, and the residual amount of fluorescent dye in the ear after a certain period of time is quantitatively determined. The residual rate can be calculated from the administered amount and the residual amount.

With respect to the residual rate of the pharmaceutical composition of the present invention, an appropriate residual rate varies according to ear diseases and/or drugs. When the retention properties of the pharmaceutical composition of the present invention are evaluated by the above method, for example, the period until the residual rate after administration becomes 50% or less is, typically 6 hours or more, preferably 12 hours or more, more preferably 1 day or more, even more preferably 2 days or more, even more preferably 3 days or more, even more preferably 5 days or more, even more preferably 6 days or more, even more preferably 7 days or more, even more preferably 10 days or more, even more preferably 12 days or more, and most preferably 14 days or more. Furthermore, the period until the residual rate after administration becomes 20% or less is, typically 6 hours or more, preferably 12 hours or more, more preferably 1 day or more, even more preferably 2 days or more, even more preferably 3 days or more, even more preferably 5 days or more, even more preferably 6 days or more, even more preferably 7 days or more, even more preferably 10 days or more, even more preferably 12 days or more, even more preferably 14 days or more, even more preferably 21 days or more, and most preferably 28 days or more. The case that shows at least the retention properties as described above is included in the case "having retention properties" as used herein, but it is not limited thereto.

With respect to the number of administrations of the pharmaceutical composition of the present invention, an appropriate number varies according to ear diseases and/or drugs. The number (frequency) is typically twice per day, preferably once per day, more preferably once every 2 days, even more preferably once every 3 days, even more preferably once every 4 days, even more preferably once every 5 days, even more preferably once every 6 days, even more preferably once every 7 days, even more preferably once every 10 days, even more preferably once every 12 days, even more preferably once every 14 days, even more preferably once every 21 days, and most preferably once every 28 days, but it is not limited thereto.

The dosage of the pharmaceutical composition is not particularly limited, as long as it can be administered to a human. It is preferable that the dosage is, an amount necessary to provide a useful contact with the tympanic membrane in an embodiment, an amount that can be administered into the tympanic cavity in an embodiment, and an amount necessary to provide a useful contact with the round window membrane in an embodiment. The dosage is typically 1 µL to 2,000 µL, preferably 10 µL to 1,500 µL, more preferably 20 µL to 1,250 µL, even more preferably 30 µL to 1,000 µL, even more preferably 50 µL to 750 µL, even more preferably 75 µL to 600 µL, even more preferably 100 µL to 500 µL, and most preferably 100 µL to 300 µL. The upper limits and the lower limits can be arbitrarily combined if desired.

The pharmaceutical composition of the present invention comprises one, or two or more drugs. The "drug" used in the present invention is typically an active ingredient needed to treat ear diseases, and preferably a small molecule compound, a nucleic acid, or a protein.

The small molecule compound is a compound having a molecular weight of less than 500. As typical small molecule compounds, there may comprise analgesics, such as acetaminophen, celecoxib, diclofenac sodium, or the like; steroids, such as dexamethasone, beclomethasone, or the like; and antibacterial drugs, such as azithromycin, amoxicillin, ofloxacin, gentamicin, or the like, but it is not limited thereto. The nucleic acid is a polymer compound in which nucleotides consisting of bases, sugars, and phosphates are linked by phosphodiester bonds. As typical nucleic acids, there may comprise a polynucleotide; an oligonucleotide; a DNA (plasmid DNA, cDNA, genomic DNA, or synthetic DNA); an RNA; a chemically modified nucleic acid; a virus, such as adenovirus, retrovirus, or the like; or the like, but it is not limited thereto. The protein means a polymer compound formed by chain polymerizing L-amino acids. As typical proteins, there may comprise an enzyme, a ligand for a target receptor, a receptor itself, an antibody, or the like, but it is not limited thereto. The protein is preferably a ligand for a target receptor, such as a drug that provides the biological activity of a heparin-binding epidermal growth factor.

As the drug that provides the biological activity of a heparin-binding epidermal growth factor used in the present invention, for example, there may comprise HB-EGF (Heparin-Binding Epidermal Growth Factor-like growth factor), human HB-EGF, recombinant human HB-EGF, or the like, but it is not limited thereto. It is typically HB-EGF, preferably human HB-EGF, and more preferably recombinant human HB-EGF.

The drug that provides the biological activity of a heparin-binding epidermal growth factor used in the present invention is typically a protein comprising an amino acid sequence having 90% or more identity with the amino acid sequence of SEQ ID NO: 1, and having a proliferation and/or a migration on keratinocytes and fibroblasts.

The drug that provides the biological activity of a heparin-binding epidermal growth factor used in the present invention is typically selected from the following proteins:
a) a protein comprising the amino acid sequence of SEQ ID NO: 1, and having a proliferation and/or a migration on keratinocytes and fibroblasts; or
b) a protein comprising an amino acid sequence in which 1 to 10 amino acids, 1 to 5 amino acids, 1 to 3 amino acids, or 1 amino acid are deleted, substituted, inserted, and/or added in the amino acid sequence of SEQ ID NO: 1, and having a proliferation and/or a migration on keratinocytes and fibroblasts.

The drug that provides the biological activity of a heparin-binding epidermal growth factor used in the present invention is typically a protein comprising the amino acid sequence of SEQ ID NO: 1, and preferably a protein consisting of the amino acid sequence of SEQ ID NO: 1.

The drug that provides the biological activity of a heparin-binding epidermal growth factor used in the present invention can be easily prepared by a person skilled in the art, based on the sequence information disclosed in the present specification, and using methods known in the art.

The heparin-binding epidermal growth factor is a protein that belongs to the EFG family (Biochimica et Biophysica Acta, 1997, 1333, F179-F199). It is known that a precursor of the heparin-binding epidermal growth factor is a membrane anchor-type protein, and a mature soluble type of the heparin-binding epidermal growth factor is released by proteolytic cleavage. As the HB-EGF used in the present invention, it is possible to select any appropriate HB-EGF or its variant or modified protein, regardless of the membrane type or soluble type or the precursor or mature type, as long as it has a proliferation and/or a migration on keratinocytes and fibroblasts. Whether or not a certain protein has the proliferation and/or a migration on keratinocytes and fibroblasts can be confirmed, for example, by a method described in "How to Choose a cell Health Assay. (Riss. T, January, 2014 tpub148)".

The pharmaceutical composition of the present invention can be used for the treatment of ear diseases, for example, typically, diseases in the outer ear or the tympanic membrane, such as acute otitis externa, chronic otitis externa, fistula auris congenita, traumatic injury of tympanic membrane, chronic tympanic membrane perforation, or the like; diseases in the middle ear, such as acute otitis media, acute mastoiditis, petrositis, otitis media with effusion, patulous Eustachian tube, chronic otitis media, cholesteatoma of the middle ear, otitic intracranial complications, adhesive otitis media, cholesterol granuloma, eosinophilic otitis media, ossicular chain discontinuity, glomus tumor, or the like; or diseases in the inner ear, such as Meniere's disease, perilymph fistula, presbyacusis, inner ear disorder caused by drug, mumps defness, benign paroxysmal positional vertigo, or the like, but it is not limited thereto. It can preferably be used for chronic tympanic membrane perforation.

Chronic tympanic membrane perforation is a disease characterized in that perforation occurs in the tympanic membrane and symptoms such as conductive hearing loss or the like are exhibited. The tympanic membrane can have a perforation caused by otitis media or trauma. In many cases, tympanic membrane perforation is closed as the tympanic membrane is reproduced, and is naturally healed. However, when the reproduction healing process of the tympanic membrane is interrupted as bacterial infection in the tympanic membrane is sustained, such as in the case of chronic otitis media or the like, the perforation is not closed, and it is diagnosed as chronic tympanic membrane perforation. Patients who suffer from hearing loss are subjected to surgical operations.

The "an effective amount" or "an amount effective for a treatment" of the drug that provides the biological activity of a heparin-binding epidermal growth factor is a dose that enhances healing of chronic tympanic membrane perforation when applied over a period of time. The lower limit of the amount of the drug that provides the biological activity of a heparin-binding epidermal growth factor that can be contained in the pharmaceutical composition of the present invention is approximately 1 ng/mL, approximately 10 ng/mL, approximately 100 ng/mL, approximately 1 µg/mL, approximately 10 µg/mL, approximately 100 µg/mL, approximately 200 µg/mL, approximately 500 µg/mL, approximately 750 µg/mL, approximately 1 mg/mL, approximately 5 mg/mL, approximately 10 mg/mL, approximately 50 mg/mL, or approximately 100 mg/mL. The upper limit of the amount of HB-EGF that can be contained in the pharmaceutical composition of the present invention is approximately 10 mg/mL, approximately 25 mg/mL, approximately 50 mg/mL, approximately 100 mg/mL, or approximately 500 mg/mL. The upper limits and the lower limits can be arbitrarily combined if desired. Additionally, in order to reduce the number of administrations, a pharmaceutical composition with a prolonged sustained-release of the drug that provides the biological activity of a heparin-binding epidermal growth factor can be provided at a higher initial concentration than that of a pharmaceutical composition for repeated administration.

In treating chronic tympanic membrane perforation, the period of contacting the drug that provides the biological activity of a heparin-binding epidermal growth factor and chronic tympanic membrane perforations is typically 1 days or more, preferably 3 days or more, more preferably 5 days or more, even more preferably 7 days or more, even more preferably 10 days or more, even more preferably 2 weeks or more, even more preferably 3 weeks or more, and most preferably 4 weeks or more.

In treating chronic tympanic membrane perforation, the sustained release properties of the drug that provides the biological activity of a heparin-binding epidermal growth factor mean, when the above-mentioned dissolution test is carried out, for example, the sustained drug release properties at 1 hour after the beginning of the dissolution test are typically 40% or less, preferably 30% or less, more preferably 20% or less, even more preferably 15% or less, and most preferably 12% or less. Further, for example, the sustained drug release properties at 6 hours after the beginning of the dissolution test are typically 70% or less, preferably 60% or less, more preferably 50% or less, even more preferably 40% or less, and most preferably 35% or less. Furthermore, for example, the sustained drug release properties at 24 hours after the beginning of the dissolution test are typically 85% or less, preferably 75% or less, even more preferably 70% or less, even more preferably 60% or less, and most preferably 55% or less.

In treating chronic tympanic membrane perforation, with respect to the residual rate of the drug that provides the biological activity of a heparin-binding epidermal growth factor in the pharmaceutical composition of the present invention, for example, the period until the residual rate after administration becomes 50% or less is typically 6 hours or more, preferably 12 hours or more, more preferably 1 day or more, even more preferably 2 days or more, even more preferably 3 days or more, even more preferably 5 days or more, even more preferably 6 days or more, even more preferably 7 days or more, even more preferably 10 days or more, even more preferably 12 days or more, and most preferably 14days or more. Furthermore, the period until the residual rate after administration becomes 20% or less is typically 6 hours or more, preferably 12 hours or more, more preferably 1 day or more, even more preferably 2 days or more, even more preferably 3 days or more, even more preferably 5 days or more, even more preferably 6 days or more, even more preferably 7 days or more, even more preferably 10 days or more, even more preferably 12 days or more, even more preferably 14 days or more, even more preferably 21 days or more, and most preferably 28 days or more.

When the drug that provides the biological activity of a heparin-binding epidermal growth factor is used, in order to suppress aggregation of the drug that provides the biological activity of a heparin-binding epidermal growth factor, structural change of the drug that provides the biological activity of a heparin-binding epidermal growth factor, or the like, pharmaceutical additives may be appropriately added alone or in combination of two or more in appropriate amounts within the range where the drug effect is exerted.

In the pharmaceutical composition of the present invention, pharmaceutical additives may be appropriately added alone or in combination of two or more in appropriate amounts, if desired, within the range where the desired effects of the present invention are exerted. As the pharmaceutical additives, for example, there may comprise an excipient such as mannitol or the like; a buffer such as PBS or the like; a pH adjuster such as diluted hydrochloric acid, sodium hydroxide, or the like; an isotonic agent; a solubilizer, an antioxidant; a preservative; a surfactant such as polysorbate 20 or the like; or the like, but it is not limited thereto.

The buffer such as PBS or the like may be added in the process of preparing a drug for the reason of stabilizing the drug. Although the composition of PBS is not particularly limited, in order to prepare PBS at pH 7.4, typically, there may comprise a method of dissolving 1.44 mg/mL of disodium hydrogen phosphate, 0.20 mg/mL of potassium chloride, 8.00 mg/mL of sodium chloride, and 0.24 mg/mL of potassium dihydrogen phosphate in purified water; a method of dissolving 1.15 mg/mL of disodium hydrogen phosphate, sodium chloride 8mg/mL, 0.2 mg/mL of potassium chloride, and 0.2 mg/mL of potassium dihydrogen phosphate in purified water; or a method of dissolving 2.9 mg/mL of disodium hydrogen phosphate dodecahydrate, 0.2 mg/mL of potassium chloride, 8.01 mg/mL of sodium chloride, and 0.2 mg/mL of potassium dihydrogen phosphate in purified water, but it is not limited thereto.

The surfactant may be added for reasons such as improving the solubility of a drug, suppressing the aggregation of a protein, or the like, but it is not limited thereto.

The method of producing the pharmaceutical composition of the present invention will be described below, but includes known methods comprising steps such as drug preparation, base dissolution, pH adjustment, filling, sterilization, drug freeze-drying, final mixing of drug and base, final sterilization, or the like.

### <Drug preparation step>

In the drug preparation step, the apparatus and means are not particularly limited, as long as the drug can be usually dissolved, suspended, or dispersed in a pharmaceutical manner. For example, there may comprise a method of dissolving, suspending, or dispersing the drug in a buffer such as PBS or the like as a solvent, but it is not limited thereto. When the drug is a protein or the like, a surfactant such as polysorbate 20 or the like may be added in order to prevent aggregation and/or to prevent adsorption to the container, but it is not limited thereto.

### <Base dissolution and pH adjustment steps>

In the base dissolution step, the apparatus and means are not particularly limited, as long as the polymer, particularly, polyvinyl caprolactam-polyvinyl acetate-polyethylene glycol graft copolymer, can be usually dissolved in a pharmaceutical manner. For example, there may comprise a method of obtaining a base solution by adding the polymer, particularly polyvinyl caprolactam-polyvinyl acetate-polyethylene glycol graft copolymer, to a solvent such as purified water or the like, and dissolving the polymer by stirring under refrigerated conditions (approximately 4°C) using a stirrer, but it is not limited thereto. When the polymer is added, an agent for adjusting complex viscosity may be added within the range where the desired effects of the present invention are achieved, if desired, but it is not limited thereto. Furthermore, a pH adjustor such as diluted hydrochloric acid, sodium hydroxide, or the like can be added to adjust the pH within the range where the desired effects of the present invention are achieved, if desired, but it is not limited thereto.

### <Filling and sterilization steps>

In the filling step and the sterilization step, the apparatus and means are not particularly limited, as long as the method used is a usually pharmaceutically acceptable filling and sterilization method. For example, there may comprise a method in which the dissolved base solution is sterilized by pressure filtration in a stirring container under refrigerated conditions, and is filled in a container such as a vial or the like, or a device or the like, but it is not limited thereto. In order to suppress variations in filling amount, an apparatus suitable for quantitative filling can be used, such as an apparatus capable of controlling the temperature in the range of 0°C to 25°C, but it is not limited thereto. The term "sterilization" used herein means killing or removing microorganisms existing in a pharmaceutical composition, a container, a device or the like. As the sterilization method, for example, there may comprise dry heat sterilization, heat sterilization, high pressure steam sterilization, chemical sterilization, radiation sterilization, or filter sterilization, but it is not limited thereto.

### <Drug freeze-drying step>

The drug solution can be frozen or freeze-dried. In the drug freeze-drying step, the apparatus and means are not particularly limited, as long as the method used is a usually pharmaceutically acceptable freeze-drying method. During freeze-drying, in order to increase the solid content concentration and maintain the properties of the cake, the freeze-drying can be carried out by adding some of the base solution components, but it is not limited thereto.

### <Step of final mixing of drug and base>

The pharmaceutical composition of the present invention can be provided as a frozen solution, a refrigerated solution, or freeze-dried product in which the drug and the base are mixed, or as a frozen solution, a refrigerated solution, or freeze-dried product of the drug, and the base solution, which are filled in separate containers, but it is not limited thereto. In the case where the drug and the base solution are provided in separate containers, the pharmaceutical composition of the present invention may be prepared, for example, by a method of adding the base solution to a vial containing the drug and mixing them, or a method of adding each from each vial containing the drug or the base solution to another empty vial and mixing them, but it is not limited thereto.

### <Final sterilization step>

The pharmaceutical composition of the present invention can comprise, in its manufacturing process, the final sterilization step, for the purpose of providing a preparation that does not contain microorganisms causing infectious diseases, or a safe pharmaceutical composition. As the sterilization method, for example, there may comprise dry heat sterilization, heat sterilization, high pressure steam sterilization, chemical sterilization, radiation sterilization, or filter sterilization, but it is not limited thereto. The preferred sterilization method is filter sterilization. In the case where the drug and the base solution are provided in separate containers, there is a possibility that the final mixing step and the final sterilization step, or the final mixing step is carried out in the medical field. It is not always necessary to mix them immediately before administration or during administration, because if the room temperature in the medical field is room temperature (1 to 30°C), it will not gelate immediately after mixing.

The container for filling the pharmaceutical composition of the present invention can be selected according to the purpose of use. The container as used herein is not particularly limited, as long as it can be sealed. For example, the container may be a vial, an ampule, a bottle-like container with large capacity, a device, or the like, but it is not limited thereto.

The pharmaceutical composition of the present invention can be delivered to a treatment site using a device. The term "device" as used herein means medical equipment or an apparatus that delivers a pharmaceutical composition to a treatment site, and can be selected according to the treatment site. The device, for example, may be a device in which a syringe (including a disposable syringe) or the like in a form of a prescribed dose is filled with a pharmaceutical composition, and equipped with a needle, a cannula, or a catheter; a micropipette; a double barrel syringe in which a pharmaceutical composition is completed by filling separate syringes or the like in a form of a prescribed dose with two types of solutions or the like, and mixing the solutions or the like in the ear; a double barrel syringe in which a pharmaceutical composition is completed by filling separate syringes or the like in a form of a prescribed dose with two types of solutions or the like, and in-line mixing the solutions or the like; a spray capable of delivering a pharmaceutical composition to the treatment site by spraying; a device capable of delivering a pharmaceutical composition by pumping; or the like, but it is not limited thereto. The preferred device is a syringe equipped with a needle. The length and thickness of the needle, cannula, or catheter can be appropriately selected according to the treatment site and a patient. In the case of an administration method in which the tympanic membrane is penetrated when locally administering the drug to or near the diseased site of the ear, it is preferred that a needle, cannula, or catheter of typically 25 to 30 gauge, and more preferably 27 to 30 gauge is selected, because a hole opens in the tympanic membrane after administration.

The device can also be used as a container. For example, the pharmaceutical composition of the present invention can be provided as a prefilled cylinder liquid preparation or an auto-injector liquid preparation in which a syringe is prefilled with a solution or the like. These eliminate the need for operations such as a dissolution operation and may enable more prompt response in the medical field.

As the material of the container, device, or the like, for example, there may be from glass, plastic, or the like, but it is not limited thereto. The container or device can be surface-treated, and can be treated with silica coating, silicone coating, sulfur treatment, various low-alkaline treatments, or the like, but it is not limited thereto.

The present invention includes use of a polyvinyl caprolactam-polyvinyl acetate-polyethylene glycol graft copolymer, as a base, for the manufacture of a pharmaceutical composition for otic administration comprising one, or two or more drugs; and use of one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof, as an agent for adjusting complex viscosity, for the manufacture of a pharmaceutical composition for otic administration comprising one, or two or more drugs, and a polyvinyl caprolactam-polyvinyl acetate-polyethylene glycol graft copolymer.

With respect to the terms "for otic administration", "base", "agent for adjusting complex viscosity", "polyvinyl caprolactam-polyvinyl acetate-polyethylene glycol graft copolymer", and "drug", which are used in the use of the present invention, the explanations therefor described in the pharmaceutical composition of the present invention can be directly applied.

With respect to the content of each component and the blending method in the use of the present invention, the explanations described in the pharmaceutical composition of the present invention can be directly applied.

### EXAMPLES

The present invention will now be further illustrated by, but is by no means limited to, the following Examples.

In the Examples below, Soluplus^{™} (registered trademark)(manufactured by BASF), which was a polyvinyl caprolactam-polyvinyl acetate-polyethylene glycol graft copolymer, Pluronic (registered trademark) F-127 (manufactured by BASF), which was a poly(oxyethylene)/poly(oxypropylene) triblock copolymer (poloxamer), trisodium citrate dihydrate (hereinafter also referred to as trisodium citrate)(manufactured by Merck), disodium hydrogen citrate 1.5 hydrate (hereinafter also referred to as disodium hydrogen citrate)(manufactured by Kanto Chemical), sodium dihydrogen citrate (manufactured by Kanto Chemical), tripotassium citrate monohydrate (hereinafter also referred to as tripotassium citrate)(manufactured by Wako Pure Chemical Industries), potassium dihydrogen citrate (manufactured by Wako Pure Chemical Industries), triammonium citrate (manufactured by Kanto Chemical), diammonium hydrogen citrate (manufactured by Wako Pure Chemical Industries), ammonium acetate (manufactured by Kanto Chemical), ammonium carbonate (manufactured by Wako Pure Chemical Industries), ammonium sulfate (manufactured by Kanto Chemical), disodium succinate (manufactured by Wako Pure Chemical Industries), sodium acetate (manufactured by Kanto Chemical), sodium carbonate (manufactured by Kanto Chemical), sodium dihydrogen phosphate dihydrate (hereinafter also referred to as sodium dihydrogen phosphate)(manufactured by Kanto Chemical), sodium tartrate dihydrate (hereinafter also referred to as sodium tartrate)(manufactured by Wako Pure Chemical Industries), sodium tetraborate decahydrate (hereinafter also referred to as sodium tetraborate)(manufactured by Merck), sodium chloride (manufactured by Kanto Chemical), sodium sulfate (manufactured by Wako Pure Chemical Industries), potassium chloride (manufactured by Kanto Chemical), calcium chloride (manufactured by Kanto Chemical), Tween 20 (manufactured by Kanto Chemical) as polysorbate 20 (hereinafter also referred to as PS20), Fluorescein isothiocyanate-dextran, average mol wt 10,000 (manufactured by Sigma-Aldrich) as FITC dextran (10 kDa), Fluorescein isothiocyanate-dextran, average mol wt 70,000 (manufactured by Sigma-Aldrich) as FITC dextran (70 kDa), Fluorescein isothiocyanate-dextran, average mol wt 150,000 (manufactured by Sigma-Aldrich) as FITC dextran (150 kDa), physiological saline (Otsuka Pharmaceutical), acetaminophen (manufactured by Yamamoto Corporation), diclofenac sodium (manufactured by FUJIFILM), and nicardipine hydrochloride (manufactured by Tokyo Chemical Industry), were used. Three types of PBS were used in the Examples. These are "1×PBS (pH 7.4)" (manufactured by Gibco)(PBS composition: disodium hydrogen phosphate heptahydrate 0.795 mg/mL, sodium chloride 9 mg/mL, and potassium dihydrogen phosphate 0.144 mg/mL); "D-PBS(-)" (manufactured by FUJIFILM)(PBS composition: disodium hydrogen phosphate 1.15 mg/mL, sodium chloride 8 mg/mL, potassium chloride 0.2 mg/mL, and potassium dihydrogen phosphate 0.2 mg/mL); and PBS prepared by dissolving 2.9 mg/mL of disodium hydrogen phosphate dodecahydrate (manufactured by Merck), 0.2 mg/mL of potassium chloride (manufactured by Merck), 8.01 mg/mL of sodium chloride (manufactured by Merck), and 0.2 mg/mL of potassium phosphate (manufactured by Merck) in purified water. In the Examples, SOLUPLUS (registered trademark) is also referred to as Sol., and Pluronic (registered trademark) is also referred to as Plu. The unit "%" used for the concentrations of Sol. and Plu. means "%(w/w)". For example, 18% of Sol. means 18%(w/w) of SOLUPLUS (registered trademark).

### Experiment 1: Examination of bases

### <Preparation Example 1-1> Preparation of Sol. base solution

After 3 g of Sol. was weighed, the Sol. was added to 7 g of purified water and dissolved at approximately 4°C to prepare a 30% Sol. base solution (Example 1). Similarly, 28% Sol. base solution (Example 2), 25% Sol. base solution (Example 3), 20% Sol. base solution (Example 4), 18% Sol. base solution (Example 5), 15% Sol. base solution (Example 6), and 10% Sol. base solution (Example 7) were prepared by varying the amount of Sol. added.

### <Preparation Example 1-2> Preparation of Plu. base solution

After 3.4 g, 3.0 g, or 2.8 g of Plu. was weighed, each Plu. was added to 16.6 mL of PBS, and dissolved at approximately 4°C to prepare a 17% Plu.-PBS base solution (Example 8), a 15% Plu.-PBS base solution (Example 9), and a 14% Plu.-PBS base solution (Example 10).

### <Experimental Example 1-1> Test for gel forming ability of base solution

With respect to the base solutions (Examples 1 to 10) prepared in Preparation Example 1-1 and Preparation Example 1-2, the gel forming ability at 25°C or 37°C was evaluated. After 0.3 mL of each base solution was dropped onto a plastic Petri dish (1000-035, manufactured by IWAKI), the dish was covered with a lid, and placed on the water surface of a water bath (TR-2A, manufactured by AS ONE Corporation) set to 25°C or 37°C for 10 minutes (N=2). After 10 minutes from the beginning of the placement, each plastic Petri dish was taken out and immediately inverted to visually observe the behavior of the base solution for 10 seconds from the inversion. The evaluation was carried out in one of the following three patterns. In Table 1, "●" means that both tests were (1), "∘" means a combination of (1) and (2), "▲" means that both tests were (2), "△" means a combination of (2) and (3), "×" means that both tests were (3), and "-" means that the test was not carried out.
(1) Neither "dropped" nor "flowed horizontally" were observed.
(2) "Dropped" and/or "flowed horizontally" was observed from 5 seconds to less than 10 seconds.
(3) "Dropped" and/or "flowed horizontally" was observed immediately after inversion to less than 5 seconds.

### <Experimental Example 1-2> Confirmation of complex viscosity of base solution

With respect to each base solution (Examples 1 to 10) prepared in Preparation Example 1-1 and Preparation Example 1-2, the complex viscosity was measured over time, using a rheometer (MCR302, manufactured by Anton Paar) equipped with a plate (MEASURING CONE CP25-2), a hood (H-PTD200), and a gap of 0.3 mm, when the temperature of the sample was increased under the conditions of a sample volume of 0.3 mL, a shear strain of 5%, an angular frequency of 50 rad/sec., a normal force of 0 N, and a torque reliability range of 1 µN·m or more (N=3). When the torque reliability range was not exceeded, it was regarded as less than the Limit of Quantitation (LOQ). When the sample temperature was 25°C, the point was regarded as the beginning point of the measurement. The temperature was increased from 25°C to 37°C at a heating rate of 1°C/10 sec., and the measurement was continued for 10 minutes after reaching 37°C. All results were shown as mean ± standard deviation.

**[Table 1]**

| | Type and concentration of base | Gel forming ability | | Complex viscosity (mPa·s) | | | |
|---|---|---|---|---|---|---|---|
| | | 25°C | 37°C | 25°C | After reaching 37°C | | |
| | | | | | 1 min. | 5 min. | 10 min. |
| Example 1 | 30% Sol. | ● | ● | 2767±100 | 7394±238 | 11156±405 | 31664±6074 |
| Example 2 | 28% Sol. | ▲ | ● | 1437±70 | 4141±349 | 6268±930 | 13656±3281 |
| Example 3 | 25% Sol. | × | ▲ | 538±13 | 1529±49 | 3510±180 | 6037±504 |
| Example 4 | 20% Sol. | - | × | 116±2 | 185±22 | 857±148 | 1740±294 |
| Example 5 | 18% Sol. | - | × | <LOQ | <LOQ | 285±44 | 614±107 |
| Example 6 | 15% Sol. | - | × | <LOQ | <LOQ | 114±18 | 148±45 |
| Example 7 | 10% Sol. | - | × | <LOQ | <LOQ | <LOQ | <LOQ |
| Example 8 | 17% Plu. | ● | ● | 48081±4276 | 59101±4494 | 60693±3624 | - |
| Example 9 | 15% Plu. | (30°C●) | ● | - | - | - | - |
| Example 10 | 14% Plu. | × | × | - | - | - | - |

The results are shown in Table 1 (Example 8 is also shown in Table 5). With respect to the gel forming ability, the 25% Sol. base solution (Example 3) did not form a gel at 25°C, and formed a gel at 37°C. The 28% Sol. base solution (Example 2) tended to form a gel gradually at 25°C, and the 30% Sol. base solution (Example 1) formed a gel at 25°C. The 10 to 20% Sol. base solutions (Examples 4 to 7) did not form a gel at 37°C. Additionally, Examples 3 and 4 formed a gel, when they were placed on the water surface of a water bath set at 37°C for 30 minutes.

With respect to the complex viscosity, at 25°C, the complex viscosity of each of the 10 to 25% Sol. base solutions (Examples 3 to 7) was less than 1,000 mPa·s, and the complex viscosity of each of the 28 to 30% Sol. base solutions (Examples 1 to 2) was 1,000 mPa·s or more. At 37°C, the complex viscosity of each of the 10 to 20% Sol. base solutions (Examples 4 to 7) was less than 2,000 mPa·s, and the complex viscosity of each of the 25 to 30% Sol. base solutions (Examples 1 to 3) was 2,000 mPa·s or more.

### Experiment 2: Examination of pharmaceutical additive acceptable to otic administration

Since it is preferable that a pharmaceutical composition for otic administration is non-irritating to the ear, pharmaceutical additives that were not irritating to the ear were further examined with respect to Sol. base solutions.

### <Preparation Example 2-1> Preparation of salt solution (target osmotic pressure 200 mOsm)

Salt solutions with an osmotic pressure of approximately 200 mOsm were prepared. More particularly, each salt described in "Type of salt added" of Table 2 was weighed in the amount described in "Amount of salt added (g)" of Table 2, respectively, and dissolved in 200 mL of purified water to prepare 20 salt solutions (Examples 11 to 30). Hereinafter, the unit "%" in the concentration of salt solutions means "%(w/v)".

### <Experimental Example 2-1> Measurement of osmotic pressure of salt solution

With respect to each salt solution prepared in Preparation Example 2-1 (Examples 11 to 30), the osmotic pressure was measured. The osmotic pressure was measured using an automatic osmometer (OM-6050, manufactured by ARKRAY) in accordance with the calibration reference value of ARKRAY and a calibration method based on

### Pharmaceutical affairs law.

**[Table 2]**

| | Type of salt added | Amount of salt added (g) | Solution concentration (%) | Osmotic pressure (mOsm) |
|---|---|---|---|---|
| Example 11 | trisodium citrate | 4.2 | 2.1 | 203 |
| Example 12 | tripotassium citrate | 4.6 | 2.3 | 205 |
| Example 13 | triammonium citrate | 3.4 | 1.7 | 202 |
| Example 14 | disodium hydrogen citrate | 4.4 | 2.2 | 200 |
| Example 15 | disodium succinate | 2.6 | 1.3 | 207 |
| Example 16 | sodium carbonate | 1.8 | 0.9 | 206 |
| Example 17 | sodium tartrate | 3.8 | 1.9 | 201 |
| Example 18 | sodium sulfate | 2.4 | 1.2 | 202 |
| Example 19 | ammonium sulfate | 2.2 | 1.1 | 200 |
| Example 20 | sodium dihydrogen citrate | 4.8 | 2.4 | 200 |
| Example 21 | potassium dihydrogen citrate | 5.2 | 2.6 | 194 |
| Example 22 | diammonium hydrogen citrate | 3.6 | 1.8 | 195 |
| Example 23 | sodium acetate | 1.8 | 0.9 | 210 |
| Example 24 | sodium dihydrogen phosphate | 3.4 | 1.7 | 200 |
| Example 25 | sodium tetraborate | 3.2 | 1.6 | 189 |
| Example 26 | sodium chloride | 1.2 | 0.6 | 195 |
| Example 27 | potassium chloride | 1.6 | 0.8 | 199 |
| Example 28 | calcium chloride | 1.8 | 0.9 | 202 |
| Example 29 | ammonium acetate | 1.6 | 0.8 | 198 |
| Example 30 | ammonium carbonate | 1.2 | 0.6 | 187 |

The results are shown in Table 2. The osmotic pressure of each salt solution was in the range of 187 to 210 mOsm.

### <Preparation Example 2-2> Preparation of 18% Sol.-salt base solution

After 20 g (or 10 g) of Sol. was weighed, the Sol. was added to 80 g (or 40 g) of each salt solution prepared in Preparation Example 2-1 and dissolved at approximately 4°C to prepare 20% Sol.-each salt base solutions. Next, 2 mL of 0.05%(v/v) PS20-containing PBS, which had been prepared by dissolving PS20 in 1×PBS (pH 7.4), was weighed, and mixed with 18 mL of each of the 20% Sol.-salt base solution to prepare 0.005% PS20-containing 18% Sol.-salt base solutions (Examples 31 to 50) described in Table 3. Similarly, purified water or 1×PBS (pH 7.4) instead of the salt solutions was used to a 0.005% PS20-containing 18% Sol. base solution (Example 51) and a 0.005% PS20-containing 18% Sol.-PBS base solution (Example 52).

### <Experimental Example 2-2> pH measurement of 18% Sol.-salt base solution

The pH of each base solution prepared in Preparation Example 2-2 (Examples 31 to 52) was measured. The measurement was carried out using a pH meter (HM-25G, manufactured by DKK-TOA Corporation). The results are shown in Table 3. The pH of each base solution was in the range of 4 to 10.5.

**[Table 3]**

| | Salt and its concentration added to 0.005% PS20-containing 18% Sol. | pH |
|---|---|---|
| Example 31 | 1.9% trisodium citrate | 6.93 |
| Example 32 | 2.1% tripotassium citrate | 6.97 |
| Example 33 | 1.5% triammonium citrate | 6.77 |
| Example 34 | 2.0% disodium hydrogen citrate | 5.05 |
| Example 35 | 1.2% disodium succinate | 6.73 |
| Example 36 | 0.8% sodium carbonate | 10.39 |
| Example 37 | 1.7% sodium tartrate | 5.57 |
| Example 38 | 1.1% sodium sulfate | 4.24 |
| Example 39 | 1.0% ammonium sulfate | 4.25 |
| Example 40 | 2.2% sodium dihydrogen citrate | 3.89 |
| Example 41 | 2.3% potassium dihydrogen citrate | 3.92 |
| Example 42 | 1.6% diammonium hydrogen citrate | 5.06 |
| Example 43 | 0.8% sodium acetate | 6.36 |
| Example 44 | 1.5% sodium dihydrogen phosphate | 4.37 |
| Example 45 | 1.4% sodium tetraborate | 9.32 |
| Example 46 | 0.5% sodium chloride | 4.18 |
| Example 47 | 0.7% potassium chloride | 4.22 |
| Example 48 | 0.8% calcium chloride | 4.11 |
| Example 49 | 0.7% ammonium acetate | 6.32 |
| Example 50 | 0.5% ammonium carbonate | 9.22 |
| Example 51 | Not added (purified water) | 4.27 |
| Example 52 | PBS | 6.07 |

### <Preparation Example 2-3> Preparation of 18% Sol.-salt base solution

Each salt described in "Salt and its concentration added to 18% Sol." of Table 4 was weighed in the amount described in "Amount of salt added (mg)" of Table 4, respectively, and dissolved in 20 mL of purified water to prepare each salt solution. After 4.4 g of Sol. was weighed, the Sol. was added to each of the salt solutions and dissolved at approximately 4°C to prepare each 18% Sol.-salt base solution described in Table 4. Similarly, purified water or 1×PBS (pH 7.4) was used instead of salt solutions to prepare an 18% Sol. base solution (Example 73) and an 18% Sol.-PBS base solution (Example 74).

### <Experimental Example 2-3> Evaluation of gel forming ability of 18% Sol.-salt base solution

With respect to each base solution prepared in Preparation Example 2-3 (Examples 53 to 74), the gel forming ability at 37°C was evaluated. The test method and the evaluation method were the same as those in Experimental Example 1-1. The results are shown in Table 4.

**[Table 4]**

| | Salt and its concentration added to 18% Sol. | Amount of salt added (mg) | Gel forming ability | |
|---|---|---|---|---|
| | | | 25°C | 37°C |
| Example 53 | 1.9% trisodium citrate | 380 | - | ● |
| Example 54 | 2.1% tripotassium citrate | 420 | - | ● |
| Example 55 | 1.5% triammonium citrate | 300 | - | ● |
| Example 56 | 2.0% disodium hydrogen citrate | 400 | - | ▲ |
| Example 57 | 1.2% disodium succinate | 240 | - | ● |
| Example 58 | 0.8% sodium carbonate | 160 | - | ● |
| Example 59 | 1.7% sodium tartrate | 340 | - | ● |
| Example 60 | 1.1% sodium sulfate | 220 | - | ● |
| Example 61 | 1.0% ammonium sulfate | 200 | - | ● |
| Example 62 | 2.2% sodium dihydrogen citrate | 440 | - | ● |
| Example 63 | 2.3% potassium dihydrogen citrate | 460 | - | × |
| Example 64 | 1.6% diammonium hydrogen citrate | 320 | - | △ |
| Example 65 | 0.8% sodium acetate | 160 | - | × |
| Example 66 | 1.5% sodium dihydrogen phosphate | 300 | - | ● |
| Example 67 | 1.4% sodium tetraborate | 280 | - | ▲ |
| Example 68 | 0.5% sodium chloride | 100 | - | × |
| Example 69 | 0.7% potassium chloride | 140 | - | × |
| Example 70 | 0.8% calcium chloride | 160 | - | × |
| Example 71 | 0.7% ammonium acetate | 140 | - | × |
| Example 72 | 0.5% ammonium carbonate | 100 | - | × |
| Example 73 | Not added (purified water) | - | - | × |
| Example 74 | PBS | - | - | × |

Although the 18% Sol. base solution (Example 73) and the 18% Sol.-PBS base solution (Example 74) did not form a gel at 37°C, some of the base solutions containing each salt solution formed a gel (Examples 53 to 62 and 66 to 67). Example 56 formed a gel, when it was placed on the water surface of a water bath set at 37°C for 30 minutes. In connection with this, since it was visually confirmed that all 18% Sol. base solutions had fluidity at 25°C in a sol state, the gel forming ability test therefor was not carried out.

### Experiment 3: Measurement of complex viscosity of 18% Sol.-salt base solution

After the pharmaceutical composition was administered into the ear, it is preferable that the viscosity increases rapidly to allow the drug to be retained in the ear. Therefore, the complex viscosity of each base solution was evaluated.

### <Experimental Example 3-1> Measurement of complex viscosity

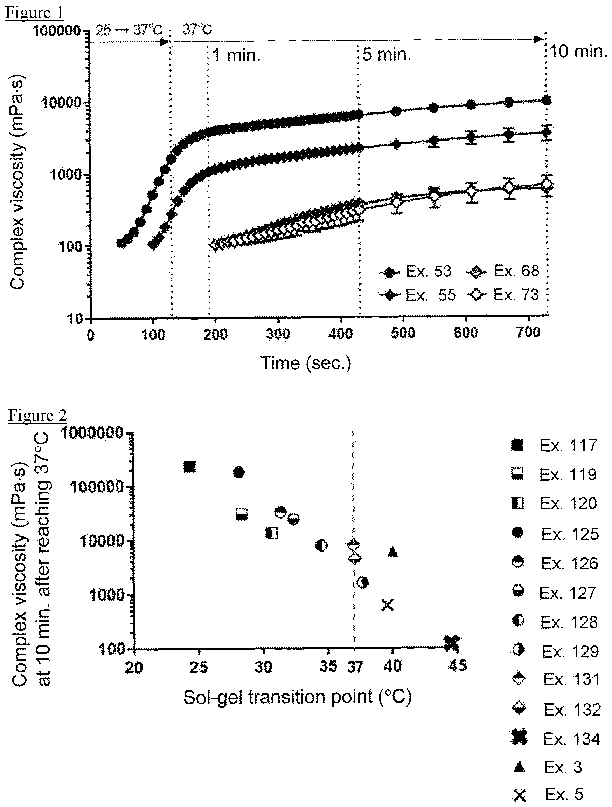

With respect to each base solution (Examples 53 to 74) prepared in Preparation Example 2-3 and the base solution (Example 8) prepared in Preparation Example 1-2, the complex viscosity was measured over time in accordance with the same test method as that in Experimental Example 1-2 (N=3). The complex viscosities of each base solution at 25°C and 30°C, and at 1 minute, 5 minutes, and 10 minutes after reaching 37°C are shown in Table 5. Further, Fig. 1 shows time courses of the complex viscosities of some base solutions. The error bar in Fig. 1 means standard error.

**[Table 5]**

| | Salt and its concentration added to 18% Sol. | Complex viscosity (mPa·s) | | | | |
|---|---|---|---|---|---|---|
| | | 25°C | 30°C | After reaching 37°C | | |
| | | | | 1 min. | 5 min. | 10 min. |
| Example 53 | 1.9% trisodium citrate | <LOQ | 128 ±6 | 3748 ±274 | 6390 ±690 | 9733 ±605 |
| Example 54 | 2.1% tripotassium citrate | <LOQ | 123 ±4 | 3253 ±157 | 3997 ±129 | - |
| Example 55 | 1.5% triammonium citrate | <LOQ | <LOQ | 1052 ±85 | 2202 ±321 | 3511 ±809 |
| Example 56 | 2.0% disodium hydrogen citrate | <LOQ | <LOQ | 1606 ±145 | 2442 ±323 | - |
| Example 57 | 1.2% disodium succinate | <LOQ | <LOQ | 1019 ±37 | 2070 ±149 | 3226 ±248 |
| Example 58 | 0.8% sodium carbonate | 108 ±3 | 109 ±1 | 1520 ±298 | 2627 ±1246 | - |
| Example 59 | 1.7% sodium tartrate | <LOQ | <LOQ | 1800 ±44 | 3011 ±195 | - |
| Example 60 | 1.1% sodium sulfate | <LOQ | 109 ±3 | 2889 ±195 | 5663 ±1050 | - |
| Example 61 | 1.0% ammonium sulfate | <LOQ | <LOQ | 811 ±9 | 1842 ±105 | - |
| Example 62 | 2.2% sodium dihydrogen citrate | 123 ±15 | 122 ±16 | 447 ±40 | 1267 ±178 | 1901 ±352 |
| Example 63 | 2.3% potassium dihydrogen citrate | <LOQ | <LOQ | 440 ±51 | 1134 ±133 | 1630 ±158 |
| Example 64 | 1.6% diammonium hydrogen citrate | 109 ±1 | 108 ±2 | 401 ±110 | 1102 ±384 | 1626 ±498 |
| Example 65 | 0.8% sodium acetate | <LOQ | <LOQ | 238 ±83 | 1058 ±383 | 1453 ±543 |
| Example 66 | 1.5% sodium dihydrogen phosphate | <LOQ | <LOQ | 617 ±49 | 1340 ±69 | - |
| Example 67 | 1.4% sodium tetraborate | <LOQ | <LOQ | 620 ±104 | 1442 ±692 | - |
| Example 68 | 0.5% sodium chloride | <LOQ | <LOQ | <LOQ | 365 ±120 | 598 ±217 |
| Example 69 | 0.7% potassium chloride | <LOQ | <LOQ | 114 ±4 | 422 ±56 | 667 ±142 |
| Example 70 | 0.8% calcium chloride | <LOQ | <LOQ | <LOQ | 291 ±37 | 429 ±56 |
| Example 71 | 0.7% ammonium acetate | <LOQ | <LOQ | <LOQ | 105 ±5 | 177 ±66 |
| Example 72 | 0.5% ammonium carbonate | <LOQ | <LOQ | 111 ±6 | 122 ±8 | 194 ±37 |
| Example 73 | Not added (purified water) | <LOQ | <LOQ | <LOQ | 305 ±89 | 665 ±221 |
| Example 74 | PBS | <LOQ | <LOQ | <LOQ | 138 ±21 | 338 ±121 |
| Example 8 | 17%Plu.-PBS | 48081 ±4276 | 57619 ±5613 | 59101 ±4494 | 60693 ±3624 | - |

At 25°C, 17% Plu.-PBS base solution carried out in Experimental Example 1-2 showed a complex viscosity exceeding 5,000 mPa·s, and the complex viscosity increased with increasing temperature. By contrast, all base solutions with Sol. as a part of the base showed a complex viscosity of approximately 100 mPa·s or less at 25°C. With increasing temperature, the complex viscosity of each base solution containing trisodium citrate, tripotassium citrate, triammonium citrate, disodium hydrogen citrate, disodium succinate, sodium carbonate, sodium tartrate, or sodium sulfate increased, and the complex viscosity of each base solution containing trisodium citrate, tripotassium citrate, or sodium sulfate became 2,000 mPa·s or more at 1 minute after reaching 37°C. The complex viscosity of each base solution containing triammonium citrate, disodium hydrogen citrate, disodium succinate, sodium carbonate, or sodium tartrate became 2,000 mPa·s or more at 5 minutes after reaching 37°C. With respect to some base solutions, the complex viscosity was measured until 10 minutes after reaching 37°C. The complex viscosities of the 18% Sol. base solution and the 18% Sol.-PBS base solution were 1,000 mPa·s or less even at 10 minutes after reaching 37°C. Further, with respect to the 18% Sol. base solutions containing sodium chloride, potassium chloride, calcium chloride, ammonium acetate, or ammonium carbonate, the complex viscosity at 10 minutes after reaching 37°C was 1,000 mPa·s or less.

### <Experimental Example 3-2> Evaluation of gel forming ability at 37°C

Among each base solution prepared in Preparation Example 2-3, with respect to base solutions containing trisodium citrate, tripotassium citrate, triammonium citrate, disodium hydrogen citrate, disodium succinate, sodium carbonate, sodium tartrate, or sodium sulfate, the gel forming ability was evaluated when they were placed at 37°C for 1 minute. The test method and the evaluation method were the same as those in Experimental Example 1-1, except that the placing time was shortened to 1 minute.

The results are shown in Table 6. The gel formation was observed in three types of 18% Sol.-1.9% trisodium citrate base solution, 18% Sol.-2.1% tripotassium citrate base solution, and 18% Sol.-1.1% sodium sulfate base solution.

**[Table 6]**

| | Salt and its concentration added to 18% Sol. | Gel forming ability |
|---|---|---|
| | | Placed at 37°C for 1 min. |
| Example 53 | 1.9% trisodium citrate | ● |
| Example 54 | 2.1% tripotassium citrate | ● |
| Example 55 | 1.5% triammonium citrate | × |
| Example 56 | 2.0% disodium hydrogen citrate | × |
| Example 57 | 1.2% disodium succinate | × |
| Example 58 | 0.8% sodium carbonate | × |
| Example 59 | 1.7% sodium tartrate | × |
| Example 60 | 1.1% sodium sulfate | ● |

### Experiment 4: Examination of salt concentration range in 18% Sol.-salt base solution

With respect to the salt concentration in Experiment 2 and Experiment 3, each solution was prepared so that the osmotic pressure was approximately 200mOsm. In this Experiment, salt solutions with an osmotic pressure of approximately 300mOsm or approximately 100 mOsm were prepared, and the gel forming ability and the complex viscosity were evaluated.

### <Preparation Example 4-1> Preparation of salt solution (target osmotic pressure 300 mOsm)

Each salt described in Table 7 was weighed in the amount described in "Amount of salt added (g)" of Table 7, respectively, and dissolved in 100 mL of purified water to prepare salt solutions of Examples 75 to 91.

### <Preparation Example 4-2> Preparation of salt solution (target osmotic pressure 100 mOsm)

Each salt described in Table 7 was weighed in the amount described in "Amount of salt added (g)" of Table 7, respectively, and dissolved in 100 mL of purified water to prepare salt solutions of Examples 92 to 95.

### <Experimental Example 4-1> Measurement of osmotic pressure of salt solution

With respect to the salt solutions of Preparation Example 4-1 and Preparation Example 4-2, the osmotic pressure was measured. The measurement method of osmotic pressure was the same as that in Experimental Example 2-1. The results are shown in Table 7. The osmotic pressure of each salt solution was in the range of 281 to 304 mOsm or in the range of 95 to 111 mOsm.

**[Table 7]**

| | Type of salt added | Amount of salt added (g) | Osmotic pressure (mOsm) |
|---|---|---|---|
| Example 75 | 3.0% trisodium citrate | 3.0 | 281 |
| Example 76 | 3.4% tripotassium citrate | 3.4 | 297 |
| Example 77 | 2.5% triammonium citrate | 2.5 | 288 |
| Example 78 | 3.3% disodium hydrogen citrate | 3.3 | 292 |
| Example 79 | 2.8% sodium tartrate | 2.8 | 289 |
| Example 80 | 1.7% ammonium sulfate | 1.7 | 294 |
| Example 81 | 3.6% sodium dihydrogen citrate | 3.6 | 291 |
| Example 82 | 4.0% potassium dihydrogen citrate | 4.0 | 288 |
| Example 83 | 2.8% diammonium hydrogen citrate | 2.8 | 292 |
| Example 84 | 1.3% sodium acetate | 1.3 | 304 |
| Example 85 | 2.6% sodium dihydrogen phosphate | 2.6 | 297 |
| Example 86 | 3.0% sodium tetraborate | 3.0 | 301 |
| Example 87 | 0.9% sodium chloride | 0.9 | 288 |
| Example 88 | 1.2% potassium chloride | 1.2 | 298 |
| Example 89 | 1.3% calcium chloride | 1.3 | 289 |
| Example 90 | 1.2% ammonium acetate | 1.2 | 295 |
| Example 91 | 1.0% ammonium carbonate | 1.0 | 294 |
| Example 92 | 1.1% trisodium citrate | 1.1 | 111 |
| Example 93 | 0.6% disodium succinate | 0.6 | 97 |
| Example 94 | 0.9% sodium tartrate | 0.9 | 100 |
| Example 95 | 0.5% ammonium sulfate | 0.5 | 95 |

### <Preparation Example 4-3> Preparation of 18% Sol.-salt base solution (target osmotic pressure 300 mOsm)

After 2 g of Sol. was weighed, the Sol. was added to 8 g of each salt solution prepared in Preparation Example 4-1 and dissolved at approximately 4°C to prepare each 20% Sol.-salt base solution. To 1.8 mL (or 1.35 mL) of each 20% Sol.-salt base solution, 0.2 mL (or 0.15 mL) of purified water was added to prepare each 18% Sol.-salt base solution (Examples 96 to 105 and 110 to 116).

### <Preparation Example 4-4> Preparation of 18% Sol.-salt base solution (target osmotic pressure 100 mOsm)

After 2 g of Sol. was weighed, the Sol. was added to 8 g of each salt solution prepared in Preparation Example 4-2 and dissolved at approximately 4°C to prepare each 20% Sol.-salt base solution. To 1.8 mL (or 1.35 mL) of each 20% Sol.-salt base solution, 0.2 mL (or 0.15 mL) of purified water was added to prepare each 18% Sol.-salt base solution (Examples 106 to 109).

### <Experimental Example 4-2> Evaluation of gel forming ability

With respect to some of the base solutions prepared in Preparation Example 4-3 and Preparation Example 4-4 (Examples 96 to 109), the presence or absence of gel formation at 37°C was evaluated (N=2). The measurement method and the evaluation method were the same as those in Experimental Example 1-1. The results are shown in Table 8.

**[Table 8]**

| | Salt and its concentration added to 18% Sol. | Gel forming ability (37°C) |
|---|---|---|
| Example 96 | 3.2% sodium dihydrogen citrate | ● |
| Example 97 | 3.6% potassium dihydrogen citrate | ● |
| Example 98 | 2.5% diammonium hydrogen citrate | ● |
| Example 99 | 1.3% sodium acetate | △ |
| Example 100 | 2.7% sodium tetraborate | ● |
| Example 101 | 0.8% sodium chloride | × |
| Example 102 | 1.1% potassium chloride | × |
| Example 103 | 1.2% calcium chloride | × |
| Example 104 | 1.1% ammonium acetate | × |
| Example 105 | 0.9% ammonium carbonate | × |
| Example 106 | 1.0% trisodium citrate | ● |
| Example 107 | 0.54% disodium succinate | × |
| Example 108 | 0.8% sodium tartrate | × |
| Example 109 | 0.45% ammonium sulfate | × |

With respect to the base solutions that were difficult to form a gel in Experimental Example 2-3, the gel formation was observed in the base solutions containing sodium dihydrogen citrate (Example 96), potassium dihydrogen citrate (Example 97), diammonium hydrogen citrate (Example 98), sodium tetraborate (Example 100), or sodium acetate (Example 99) by increasing the salt concentration to near isotonic. By contrast, the gel formation was not observed in the base solutions containing sodium chloride (Example 101), potassium chloride (Example 102), calcium chloride (Example 103), ammonium acetate (Example 104), ammonium carbonate (Example 105), even if the salt concentration was increased to isotonic. Additionally, the gel formation was observed in the base solution containing trisodium citrate (Example 106), even if the salt concentration was decreased.

### <Experimental Example 4-3> Measurement of complex viscosity

With respect to the base solutions prepared in Preparation Example 4-3 (Examples 96 to 105 and 110 to 116), the complex viscosity was measured (N=3). The test method was the same as that in Experimental Example 1-2, except for the measurement time after reaching 37°C.

**[Table 9]**

| | Salt and its concentration added to 18% Sol. | Complex viscosity (mPa·s) | |
|---|---|---|---|
| | | 25°C | 5 min. after reaching 37°C |
| Example96 | 3.2% sodium dihydrogen citrate | <LOQ | 5219±1567 |
| Example97 | 3.6% potassium dihydrogen citrate | 125±9 | 4720±700 |
| Example98 | 2.5% diammonium hydrogen citrate | <LOQ | 5351±109 |
| Example99 | 1.3% sodium acetate | 112±8 | 2043±268 |
| Example 100 | 2.7% sodium tetraborate | <LOQ | 2101±387 |
| Example 101 | 0.8% sodium chloride | <LOQ | 857±146 |
| Example 102 | 1.1% potassium chloride | <LOQ | 658±257 |
| Example 103 | 1.2% calcium chloride | 105±4 | 429±41 |
| Example 104 | 1.1% ammonium acetate | <LOQ | 541±118 |
| Example 105 | 0.9% ammonium carbonate | 150±25 | 1210±311 |
| Example110 | 2.7% trisodium citrate | 386±60 | 27709±15743 |
| Example111 | 3.1% tripotassium citrate | 216±10 | 44865±10156 |
| Example112 | 2.3% triammonium citrate | | 7556±2070 |
| Example113 | 3.0% disodium hydrogen citrate | 121±10 | 15195±9494 |
| Example114 | 2.5% sodium tartrate | 113±11 | 7088±750 |
| Example115 | 1.5% ammonium sulfate | 107±9 | 6876±1110 |
| Example116 | 2.3% sodium dihydrogen phosphate | <LOQ | 4049±903 |

The results are shown in Table 9. With respect to the base solutions that increased the complex viscosity relatively quickly, the complex viscosities at 25°C, and at 5 minutes after reaching 37°C are shown in Table 9.

The complex viscosity at 25°C was less than 500 mPa·s in all of Examples 96 to 105 and 110 to 116, and the complex viscosity at 5 minutes after reaching 37°C was 1,650 mPa·s or less in the base solutions other than those containing sodium chloride, potassium chloride, calcium chloride, ammonium acetate, or ammonium carbonate. The base solutions containing trisodium citrate or tripotassium citrate showed particularly high complex viscosities at 5 minutes after reaching 37°C. With respect to the base solutions containing sodium chloride, potassium chloride, calcium chloride, ammonium acetate, or ammonium carbonate, the complex viscosity was measured until 10 minutes after reaching 37°C, but all base solutions showed 3,000 mPa·s or less.

### Experiment 5: Examination of Sol. concentration range

The gel forming ability, the complex viscosity, and the sol-gel transition point of base solutions were evaluated when the Sol. concentration was changed.

### <Preparation Example 5> Preparation of Sol.-trisodium citrate base solution at each concentration

The base solutions described in Table 10 (Examples 117 to 134) were prepared. More particularly, after 1.0%, 1.9%, or 2.7%(w/v) of a trisodium citrate solution was prepared, Sol. was weighed and added to each solution to become the weight ratios described in Table 10, and dissolved at approximately 4°C to prepare Sol.-trisodium citrate base solutions at each concentration.

### <Experimental Example 5-1> Evaluation of gel forming ability

With respect to the base solutions prepared in Preparation Example 5, the gel formation ability at 37°C was evaluated (N=2). With respect to some of the base solutions, the gel forming ability at 25°C was also evaluated. The test method was the same as that in Experimental Example 1-1.

### <Experimental Example 5-2> Measurement of complex viscosity

With respect to the base solutions prepared in Preparation Example 5, the complex viscosity was measured. The test method and the like were the same as those in Experimental Example 1-2.

### <Experimental Example 5-3> Measurement of sol-gel transition point

With respect to some of the base solutions prepared in Preparation Example 5 and Preparation Example 1-1, the sol-gel transition point was measured (N=3). With respect to the samples in which the sol-gel transition point was measured, a 0.05% PS20-containing PBS solution was added to the samples before the measurement so that the PS20 concentration in the base solution was 0.005%. The sol-gel transition point was measured using a rheometer (MCR302, manufactured by Anton Paar) under the following conditions:
Plate: MEASURING CONE CP25-2,
Gap: 0.3 mm,
Sample volume: 0.3 mL,
Shear strain: 5%,
Angular frequency: 50 rad/sec.,
Beginning of measurement: Sample temperature of 25°C (or 10°C), and
Temperature increased: 1°C per 10 seconds, up to 45°C.

**[Table 10]**

| | Sol. (%) | Trisodium citrate (%) | Gel forming ability | | Sol-gel transition point (°C) | Complex viscosity (mPa·s) | |
|---|---|---|---|---|---|---|---|
| | | | 25°C | 37°C | | 25°C | 10 min. after reaching 37°C |
| Example 117 | 25 | 2.7 | ● | ● | 24.4±0.5 | 5612±259 | 235430±31956 |
| Example 118 | 20 | | ▲ | ● | - | 586±76 | 86238±37923 |
| Example 119 | 18 | | - | ● | 28.4±0.1 | 213±4 | 30637±1244 |
| Example 120 | 15 | | - | ● | 30.7±0.5 | 105±8 | 13515±406 |
| Example 121 | 10 | | - | ● | - | <LOQ | 2416±844 |
| Example 122 | 9 | | - | - | - | <LOQ | 2451±1970 |
| Example 123 | 8 | | - | ● | - | <LOQ | 1425±547 |
| Example 124 | 5 | | - | △ | - | <LOQ | 190±9 |
| Example 125 | 25 | 1.9 | ▲ | ● | 28.2±0.0 | 1566±47 | 180213±13156 |
| Example 126 | 20 | | - | ● | 31.4±0.1 | 162±9 | 32922±11899 |
| Example 127 | 18 | | - | ● | 32.4±0.1 | <LOQ | 24714±7670 |
| Example 128 | 15 | | - | ● | 34.5±0.5 | <LOQ | 7814±1896 |
| Example 129 | 10 | | - | ● | 37.7±0.0 | <LOQ | 1601±303 |
| Example 130 | 25 | 1.0 | × | ● | - | 711±55 | 98685±52260 |
| Example 131 | 20 | | - | ● | 37.0±0.1 | 118±12 | 7890±95 |
| Example 132 | 18 | | - | ● | 37.1±0.1 | 98±0 | 4450±948 |
| Example 133 | 15 | | - | ▲ | - | <LOQ | 950±144 |
| Example 134 | 10 | | - | × | 44.5±0.5 | <LOQ | 120±6 |
| Example 3 | 25 | - | × | ▲ | 40.0±1.3 | 538±13 | 6037±504 |
| Example 5 | 18 | - | - | × | 39.6±0.2 | <LOQ | 614±107 |

The results are shown in Table 10. A gel was not formed at 37°C in the base solution in which the Sol. concentration was 10%, and the trisodium citrate concentration was 1.0% (Example 134). In the base solution in which the Sol. concentration was increased to 15% (Example 133), the gel formation was observed, but the complex viscosity was approximately 950 mPa·s at 10 minutes after reaching 37°C.

The relationship between the complex viscosities in Experimental Example 5-2 at 10 minutes after reaching 37°C and the sol-gel transition points in Experimental Example 5-3 are shown in Fig. 2. The complex viscosity and the sol-gel transition point changed depending on the Sol. concentration and the trisodium citrate concentration in each base solution.

### Experiment 6: Complex viscosity of pharmaceutical composition containing human HB-EGF

The effect of a drug on the complex viscosity of a pharmaceutical composition was evaluated. More particularly, the complex viscosity when human HB-EGF as the drug was contained in an 18% Sol. base solution or an 18% Sol.-1.9% trisodium citrate base solution was evaluated.

### <Preparation Example 6-1> Preparation of 18% Sol. base solution and 18% Sol. solution containing HB-EGF

After 2g of Sol. was weighed, the Sol. was added to 8g of purified water, and dissolved at approximately 4°C to prepare a 20% Sol. base solution. To 1.8 mL of the solution, 0.2 mL of purified water or 0.2 mL of an HB-EGF solution (a PS20/PBS solution containing 150 µg/mL of human HB-EGF and 0.05% PS20, manufactured by AGC Inc.) was added to prepare an 18% Sol. base solution (Example 135) and a 15 µg/mL HB-EGF-containing 18% Sol. solution (Example 136). The PBS used in this preparation was prepared by dissolving 2.9 mg/mL of disodium hydrogen phosphate dodecahydrate, 0.2 mg/mL of potassium chloride, 8.01 mg/mL of sodium chloride, and 0.2 mg/mL of potassium dihydrogen phosphate in purified water.

### <Preparation Example 6-2> Preparation of 18% Sol.-1.9% trisodium citrate base solution and 18% Sol.-1.9% trisodium citrate solution containing HB-EGF

After 168 mg of trisodium citrate was dissolved in 8g of purified water, 2g of Sol. was weighed and added thereto, and dissolved at approximately 4°C to prepare a 20% Sol.-2.1% trisodium citrate base solution. To 1.8 mL of the solution, 0.2 mL of purified water or 0.2 mL of the HB-EGF solution was added to prepare an 18% Sol.-1.9% trisodium citrate base solution (Example 137) and a 15µg/mL HB-EGF-containing 18% Sol.-1.9% trisodium citrate solution (Example 138).

### <Experimental Example 6-1> Measurement of complex viscosity

With respect to each base solution or each solution prepared in Preparation Example 6-1 and Preparation Example 6-2, the complex viscosity was measured. The test method was the same as that in Experimental Example 1-2. The results are shown in Table 11.

**[Table 11]**

| | Base solution or solution | Complex viscosity (mPa·s) | | | | |
|---|---|---|---|---|---|---|
| | | 25°C | 30°C | After reaching 37°C | | |
| | | | | 1 min. | 5 min. | 10 min. |
| Example 135 | 18% Sol. | <LOQ | <LOQ | 135 ± 27 | 506 ± 147 | 949 ± 144 |
| Example 136 | 15 µg/mL HB-EGF-containing 18%Sol. | <LOQ | <LOQ | 120 ± 4 | 360 ± 41 | 801 ± 56 |
| Example 137 | 18% Sol.-1.9% trisodium citrate | 122 ± 20 | 240 ± 8 | 5552 ± 182 | 8706 ± 157 | 13668 ± 846 |
| Example 138 | 15 µg/mL HB-EGF-containing 18% Sol. -1.9% trisodium citrate | 136 ± 5 | 414 ± 41 | 6043 ± 609 | 8591 ± 332 | 13934 ± 1002 |

No significant difference was found in the complex viscosity, both between the 18% Sol. base solution (Example 135) and the 15µg/mL HB-EGF-containing 18% Sol. solution (Example 136), and between the 18% Sol.-1.9% trisodium citrate base solution (Example 137) and the 15µg/mL HB-EGF-containing 18% Sol.-1.9% trisodium citrate solution (Example 138).

### <Experiment 7> Evaluation of sustained drug release of pharmaceutical composition for otic administration

With respect to the Sol. base solutions or the Sol.-salt base solutions, the sustained drug release was evaluated using a plurality of model drugs. As the model drugs, FITC dextran (10 kDa, 70 kDa, and 150 kDa)(hereinafter also referred to as FITC-DEX) as a drug model of proteins, acetaminophen and diclofenac sodium as drug models of acidic small molecules with different partition coefficients, and nicardipine hydrochloride as a drug model of basic small molecules were used.

### <Preparation Example 7-1> Preparation of FITC-DEX (10 kDa)-containing solution

A 1%(w/v) FITC-DEX-containing solution was prepared by dissolving 30 mg of FITC-DEX (10 kDa) in a 0.05%(v/v) PS20-containing PBS solution and adjusting the volume to 3 mL.

Further, a 0.1%(w/v) FITC-DEX-containing 20% Sol.-1.9% trisodium citrate solution (Example 143) by mixing 200 µL of 1%(w/v) FITC-DEX into 1800 µL of 22.2% Sol.-2.1%(w/v) trisodium citrate base solution. Similarly, 0. 1%(w/v) FITC-DEX (10 kDa)-containing solutions described in Examples 139 to 142 and 144 of Table 12 were prepared by mixing 200 µL of 1%(w/v) FITC-DEX into 1800 µL of 22.2% Sol.-1.1% trisodium citrate base solution, 20% Sol.-2.1% trisodium citrate base solution, 16.7% Sol.-2.1% trisodium citrate base solution, 11.1% Sol.-2.1% trisodium citrate base solution, or 1×PBS (pH 7.4).

A 0.1%(w/v) FITC-DEX-containing 18% Sol. solution (Example 145) was prepared by mixing 150 µL of 1%(w/v) FITC-DEX into 1350 µL of 20% Sol. base solution prepared by a method similar to that in Preparation Example 2-2.

With respect to the salt solutions containing triammonium citrate, disodium succinate, or sodium dihydrogen citrate prepared in Preparation Example 2-1, 20% Sol.-base solutions of each salt solution containing 20%(w/w) Sol. were prepared, respectively. 0.1%(w/v) FITC-DEX-containing 18% Sol.-each salt solutions (Examples 146 to 148) were prepared by mixing 150 µL of 1%(w/v) FITC-DEX into 1350 µL of each of the base solutions.

After 18.9 g of Plu. was weighed, the Plu. was added to 81.1 g of PBS, and dissolved at approximately 4°C to prepare an 18.9% Plu.-PBS base solution. A 0. 1%(w/v) FITC-DEX-containing 17% Plu.-PBS solution (Example 149) was prepared by mixing 300 µL of 1%(w/v) FITC-DEX into 2700 µL of the base solution.

### <Experimental Example 7-1> Dissolution test of FITC-DEX (10 kDa)-containing solution

With respect to each solution prepared in Preparation Example 7-1, a dissolution test was carried out (N=2). The dissolution test was carried out by putting 2 mL of 1×PBS (pH 7.4) into the lower side of each well of Transwell (6 well, 12 mm diameter inserts, 0.4 µm pore size, manufacturing by Corning), heating the Transwell in a CHROMATO Chamber (M-600FN, manufactured by TAITEC) set at 37°C, adding 0.5 mL of each solution prepared in Preparation Example 7-1 to the upper side of each well, and shaking the Transwell at 37°C ± 2°C and 100 rpm. At 1 hour, 2 hours, 4 hours, 6 hours, and 24 hours after the beginning of the test, 0.5 mL of aliquot was sampled from the lower side of each well, and 0.5 mL of PBS was added to the lower side of each well. The FITC concentration in each sample liquid was measured using a fluorescent plate reader (Spectra Max i3x, manufactured by Molecular Devices) at an excitation wavelength of 494 nm and a measurement wavelength of 522 nm.

**[Table 12]**

| | FITC-DEX (10 kDa)-containing solution | Dissolution rate (%) | | |
|---|---|---|---|---|
| | | 1 hour | 6 hours | 24 hours |
| Example 139 | PBS (pH 7.4) | 76.3 | 96.4 | 99.8 |
| Example 140 | 10% Sol.-1.9% trisodium citrate | 9.5 | 28.0 | 50.5 |
| Example 141 | 15% Sol.-1.9% trisodium citrate | 6.3 | 19.2 | 35.5 |
| Example 142 | 18% Sol.-1.9% trisodium citrate | 7.6 | 19.3 | 38.1 |
| Example 143 | 20% Sol.-1.9% trisodium citrate | 5.5 | 15.5 | 28.4 |
| Example 144 | 20% Sol.-1.0% trisodium citrate | 4.4 | 18.5 | 36.2 |
| Example 145 | 18% Sol. | 11.5 | 32.5 | 49.6 |
| Example 146 | 18% Sol.-1.5% triammonium citrate | 8.3 | 28.6 | 46.6 |
| Example 147 | 18% Sol.-1.2% disodium succinate | 10.5 | 27.7 | 50.4 |
| Example 148 | 18% Sol.-2.2% sodium dihydrogen citrate | 3.2 | 9.4 | 15.8 |
| Example 149 | 17% Plu.-PBS | 14.6 | 48.9 | 59.7 |

The results are shown in Table 12. With respect to all solutions containing polymer (Examples 140 to 149), FITC-DEX (10 kDa) was slowly released. With respect to the solutions containing Sol. (Examples 140 to 143), with increasing the Sol. concentration, the dissolution rate at 1 hour after the beginning of the test tended to decrease, but the difference in dissolution rate was less than 10%. The difference in dissolution rate due to the difference in the concentration of trisodium citrate (Examples 143 and 144) was also less than 10%. Although there are some differences in dissolution rate depending on the type of salt solutions, all of the 18% Sol.-salt base solutions (Examples 142 and 146 to 148) showed sustained release, and the dissolution rates thereof were slower than that of the 17% Plu.-PBS base solution (Example 149).

### <Preparation Example 7-2> Preparation of FITC-DEX (70 kDa)-containing solution

50 mg of FITC-DEX (70 kDa) was dissolved in D-PBS to prepare 5 mL of 1%(w/v) FITC-DEX. A 0.1%(w/v) FITC-DEX-containing PBS solution (Example 150) was prepared by mixing 200 µL of 1%(w/v) FITC-DEX into 1800 µL of D-PBS.

After 56.7 g of Plu. was weighed, the Plu. was added to 243.3 g of D-PBS, and dissolved at approximately 4°C to prepare an 18.9% Plu.-PBS base solution. A 0.1%(w/v) FITC-DEX-containing 17% Plu.-PBS solution (Example 151) was prepared by mixing 200 µL of 1%(w/v) FITC-DEX into 1800 µL of the base solution.

After 60.0 g of Sol. was weighed, the Sol. was added to 240.0 g of purified water, and dissolved at approximately 4°C to prepare a 20% Sol. base solution. A 0.1%(w/v) FITC-DEX-containing 18% Sol. solution (Example 152) was prepared by mixing 200 µL of 1%(w/v) FITC-DEX into 1800 µL of the base solution.

With respect to the salt solutions containing trisodium citrate or triammonium citrate prepared in Preparation Example 2-1, base solutions of 20% Sol.-each salt solution were prepared, respectively. 0.1%(w/v) FITC-DEX-containing 18% Sol.-each salt solutions (Examples 153 and 154) were prepared by mixing 200 µL of 1%(w/v) FITC-DEX into 1800 µL of each of the base solutions.

### <Experimental Example 7-2> Dissolution test of FITC-DEX (70 kDa)-containing solution

With respect to each solution prepared in Preparation Example 7-2, the dissolution test was carried out (N=2). The test conditions were the same as those of Experimental Example 7-1, except that D-PBS was used as the dissolution test solution. The FITC concentration in each sample liquid was measured using a fluorescent plate reader (Infinite M1000PRO, manufactured by TECAN) at an excitation wavelength of 494 nm and a measurement wavelength of 522 nm.

**[Table 13]**

| | FITC-DEX (70 kDa)-containing solution | Dissolution rate (%) | | |
|---|---|---|---|---|
| | | 1 hour | 6 hours | 24 hours |
| Example 150 | PBS | 42.6 | 77.9 | 83.8 |
| Example 151 | 17% Plu.-PBS | 15.2 | 58.3 | 69.6 |
| Example 152 | 18% Sol. | 6.1 | 15.3 | 28.5 |
| Example 153 | 18% Sol.-1.9% trisodium citrate | 5.8 | 13.4 | 24.6 |
| Example 154 | 18% Sol.-1.5% triammonium citrate | 5.0 | 11.0 | 17.4 |

The results are shown in Table 13. As similar to FITC-DEX (10 kDa), with respect to FITC-DEX (70 kDa), all solutions containing 18% Sol. (Examples 152 to 154) showed sustained release, and the dissolution rates thereof were slower than that of the 17% Plu.-PBS solution (Example 151).

### <Preparation Example 7-3> Preparation of FITC-DEX (150 kDa)-containing solution

50 mg of FITC-DEX (150 kDa) was dissolved in D-PBS, and the volume was adjusted to 5 mL to prepare 1%(w/v) FITC-DEX. A 0.1%(w/v) FITC-DEX-containing PBS solution (Example 155) was prepared by mixing 200 µL of 1%(w/v) FITC-DEX into 1800 µL of D-PBS.

A 0.1%(w/v) FITC-DEX-containing 17% Plu.-PBS solution (Example 156) was prepared by mixing 200 µL of 1%(w/v) FITC-DEX into 1800 µL of an 18.9% Plu.-PBS base solution prepared by a method similar to that in Preparation Example 7-2.

A 0.1%(w/v) FITC-DEX-containing 18% Sol. solution (Example 157) was prepared by mixing 200 µL of 1%(w/v) FITC-DEX into 1800 µL of 20% Sol. base solution prepared by a method similar to that in Preparation Example 7-2.

With respect to the salt solutions containing trisodium citrate or triammonium citrate prepared in Preparation Example 2-1, base solutions of 20% Sol.-each salt solution were prepared, respectively. 0.1%(w/v) FITC-DEX-containing 18% Sol.-each salt solutions (Examples 158 and 159) were prepared by mixing 200 µL of 1%(w/v) FITC-DEX into 1800 µL of each of the base solutions.

### <Experimental Example 7-3> Dissolution test of FITC-DEX (150 kDa)-containing solution

With respect to each solution prepared in Preparation Example 7-3, the dissolution test was carried out (N=2). The test conditions were the same as those of Experimental Example 7-2. The FITC concentration in each sample liquid was measured using a fluorescent plate reader (Infinite M1000PRO, manufactured by TECAN) at an excitation wavelength of 494 nm and a measurement wavelength of 522 nm.

**[Table 14]**

| | FITC-DEX (150 kDa)-containing solution | Dissolution rate (%) | | |
|---|---|---|---|---|
| | | 1 hour | 6 hours | 24 hours |
| Example 155 | PBS | 26.7 | 74.6 | 88.5 |
| Example 156 | 17% Plu.-PBS | 15.9 | 63.0 | 79.0 |
| Example 157 | 18% Sol. | 8.4 | 20.5 | 37.3 |
| Example 158 | 18% Sol.-1.9% trisodium citrate | 5.0 | 11.5 | 19.6 |
| Example 159 | 18% SoL-1.5% triammonium citrate | 7.7 | 17.5 | 29.0 |

The results are shown in Table 14. As similar to FITC-DEX (10 kDa) and FITC-DEX (70 kDa), all solutions containing 18% Sol. (Examples 157 to 159) showed sustained release, and the dissolution rates thereof were slower than that of the 17% Plu.-PBS solution (Example 156).

### <Preparation Example 7-4> Preparation of acetaminophen-containing solution

100 mg of acetaminophen was dissolved in a 0.05%(v/v) PS20-containing PBS solution, and the volume was adjusted to 10 mL to prepare 1%(w/v) acetaminophen. A 0.1%(w/v) acetaminophen-containing PBS solution (Example 160) was prepared by mixing 120 µL of 1%(w/v) acetaminophen into 1080 µL of PBS.

A 0.1% acetaminophen-containing 17% Plu.-PBS solution (Example 161) was prepared by mixing 300 µL of 1%(w/v) acetaminophen into 2700 µL of the 18.9% Plu.-PBS base solution prepared in Preparation Example 7-2.

A 0.1%(w/v) acetaminophen-containing 18% Sol. solution (Example 162) was prepared by mixing 120 µL of 1%(w/v) acetaminophen into 1080 µL of a 20% Sol. base solution prepared by a method similar to that in Preparation Example 2-2.

With respect to the salt solutions containing trisodium citrate, triammonium citrate, disodium succinate, or sodium dihydrogen citrate prepared in Preparation Example 2-1, base solutions of 20% Sol.-each salt solution were prepared, respectively. 0.1%(w/v) acetaminophen-containing 18% Sol.-each salt solutions (Examples 163 to 166) were prepared by mixing 150 µL of 1%(w/v) acetaminophen into 1080 µL of each of the base solutions.

### <Experimental Example 7-4> Dissolution test of acetaminophen-containing solution

With respect to each solution prepared in Preparation Example 7-4, the dissolution test was carried out (N=2). The test conditions were the same as those in Experimental Example 7-1. The acetaminophen concentration in each sample liquid was measured using an HPLC (ESPRIMO D583/N, manufactured by Waters) at a measurement wavelength of 254 nm.

**[Table 15]**

| | Acetaminophen-containing solution | Dissolution rate (%) | | |
|---|---|---|---|---|
| | | 1 hour | 6 hours | 24 hours |
| Example 160 | PBS | 71.8 | 78.8 | 89.0 |
| Example 161 | 17% Plu.-PBS | 28.1 | 82.1 | 100.0 |
| Example 162 | 18% Sol. | 11.1 | 30.8 | 42.6 |
| Example 163 | 18% Sol.-1.9% trisodium citrate | 8.5 | 24.0 | 36.8 |
| Example 164 | 18% Sol.-1.5% triammonium citrate | 8.8 | 23.5 | 37.1 |
| Example 165 | 18% Sol.-1.2% disodium succinate | 8.8 | 28.1 | 41.1 |
| Example 166 | 18% Sol.-2.2% sodium dihydrogen citrate | 10.7 | 29.0 | 41.0 |

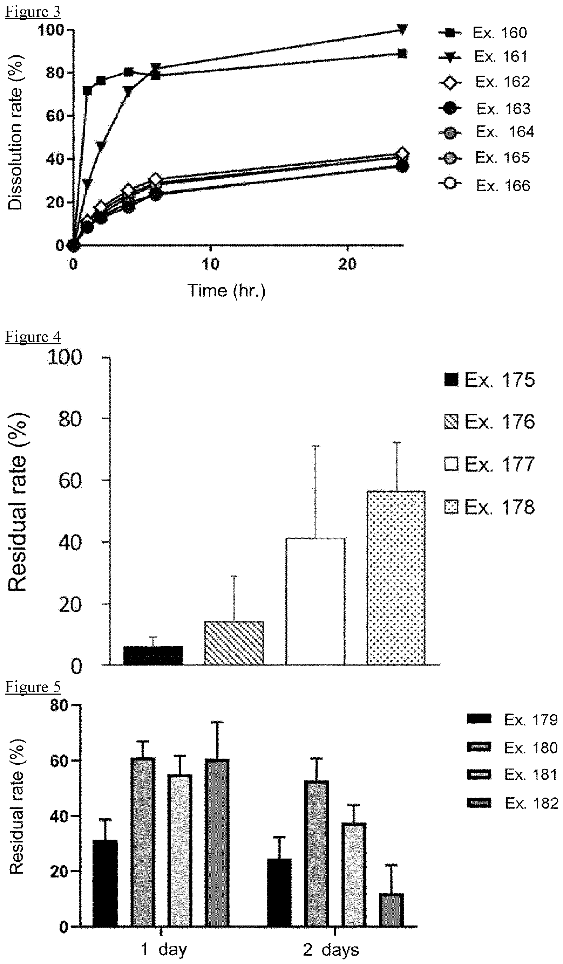

The results are shown in Table 15 and Fig. 3. With respect to all solutions containing polymer (Examples 161 to 166), acetaminophen was slowly released. The dissolution rates of all solutions containing 18% Sol. (Examples 162 to 166) were slower than that of the 17% Plu.-PBS solution (Example 161).

### <Preparation Example 7-5> Preparation of diclofenac sodium-containing solution

50 mg of diclofenac sodium was dissolved in D-PBS, and the volume was adjusted to 5 mL to prepare 1%(w/v) diclofenac sodium. A 0. 1%(w/v) diclofenac sodium-containing PBS solution (Example 167) was prepared by mixing 200 µL of 1%(w/v) diclofenac sodium into 1800 µL of D-PBS.

A 0.1%(w/v) diclofenac sodium-containing 17% Plu.-PBS solution (Example 168) was prepared by mixing 200 µL of 1%(w/v) diclofenac sodium into 1800 µL of an 18.9% Plu.-PBS base solution prepared by a method similar to that in Preparation Example 7-2.

A 0.1%(w/v) diclofenac sodium-containing 18% Sol. solution (Example 169) was prepared by mixing 200 µL of 1%(w/v) diclofenac sodium into 1800 µL of a 20% Sol. base solution prepared by a method similar to that in Preparation Example 7-2.

A 20% Sol.-2.1% trisodium citrate base solution was prepared using the salt solution containing trisodium citrate prepared in Preparation Example 2-1. A 0.1%(w/v) diclofenac sodium-containing 18% Sol.-1.9% trisodium citrate solution (Example 170) was prepared by mixing 200 µL of 1%(w/v) diclofenac sodium into 1800 µL of the base solution.

### <Experimental Example 7-5> Dissolution test of diclofenac sodium-containing solution

With respect to each solution prepared in Preparation Example 7-5, the dissolution test was carried out (N=2). The test conditions were the same as those in Experimental Example 7-2. The diclofenac sodium concentration in each sample liquid was measured using an HPLC (Waters Alliance e2695, manufactured by Waters) at a measurement wavelength of 240 nm.

**[Table 16]**

| | Diclofenac sodium-containing solution | Dissolution rate (%) | | |
|---|---|---|---|---|
| | | 1 hour | 6 hours | 24 hours |
| Example 167 | PBS | 76.7 | 89.1 | 93.1 |
| Example 168 | 17% Plu.-PBS | 15.0 | 66.8 | 91.8 |
| Example 169 | 18% Sol. | 2.8 | 7.4 | 11.6 |
| Example 170 | 18% Sol.-1.9% trisodium citrate | 2.5 | 6.3 | 9.6 |

The results are shown in Table 16. With respect to all solutions containing polymer (Examples 168 to 170), diclofenac sodium was slowly released. The dissolution rates of all solutions containing 18% Sol. (Examples 169 and 170) were slower than that of the 17% Plu.-PBS solution (Example 168).

### <Preparation Example 7-6> Preparation of nicardipine hydrochloride-containing solution

25 mg of nicardipine hydrochloride was dissolved in purified water, and the volume was adjusted to 5 mL to prepare 0.5%(w/v) nicardipine hydrochloride. A 0.05%(w/v) nicardipine hydrochloride-containing physiological saline (Example 171) was prepared by mixing 200 µL of 0.5%(w/v) nicardipine hydrochloride into 1800 µL of physiological saline.

A 0.05%(w/v) nicardipine hydrochloride-containing 17% Plu.-PBS solution (Example 172) was prepared by mixing 200 µL of 0.5%(w/v) nicardipine hydrochloride into 1800 µL of an 18.9% Plu.-PBS base solution prepared by a method similar to that in Preparation Example 7-2.

A 0.05%(w/v) nicardipine hydrochloride-containing 18% Sol. solution (Example 173) was prepared by mixing 200 µL of 0.5%(w/v) nicardipine hydrochloride into 1800 µL of 20% Sol. base solution prepared by a method similar to that in Preparation Example 7-2.

A 20% Sol.-2.1% trisodium citrate base solution was prepared using the salt solution containing trisodium citrate prepared in Preparation Example 2-1. A 0.05%(w/v) nicardipine hydrochloride-containing 18% Sol.-1.9% trisodium citrate solution (Example 174) was prepared by mixing 200 µL of 0.5%(w/v) nicardipine hydrochloride into 1800 µL of the base solution.

### <Experimental Example 7-6> Dissolution test of nicardipine hydrochloride-containing solution

With respect to each solution prepared in Preparation Example 7-6, the dissolution test was carried out (N=2). The test conditions were the same as those in Experimental Example 7-1, except that physiological saline was used as the dissolution test solution. The nicardipine hydrochloride concentration in each sample liquid was measured using an HPLC (Waters Alliance e2695, manufactured by Waters) at a measurement wavelength of 254 nm.

**[Table 17]**

| | Nicardipine hydrochloride-containing solution | Dissolution rate (%) | | |
|---|---|---|---|---|
| | | 1 hour | 6 hours | 24 hours |
| Example 171 | Physiological saline | 77.0 | 84.7 | 86.2 |
| Example 172 | 17% Plu.-PBS | 15.9 | 77.5 | 94.2 |
| Example 173 | 18% Sol. | 4.6 | 7.7 | 10.4 |
| Example 174 | 18% Sol.-1.9% trisodium citrate | 1.1 | 1.4 | 1.7 |

The results are shown in Table 17. With respect to all solutions containing polymer (Examples 172 to 174), nicardipine hydrochloride was slowly released. The dissolution rates of all solutions containing 18% Sol. (Examples 173 and 174) were slower than that of the 17% Plu.-PBS solution (Example 172).

### Experiment 8: Evaluation of retention properties in ear using fluorescent dye

Retention properties in the ear were evaluated using each base solution containing Plu. or Sol. as the base and FITC-DEX (10 kDa) as the model drug.

### <Preparation Example 8> Preparation of FITC-DEX (10 kDa)-containing solution

14 mg of FITC-DEX (10 kDa) was dissolved in purified water, and the volume was adjusted to 1 mL to prepare a 1.4%(w/v) FITC-DEX solution. A 0.14%(w/v) FIT-DEX-containing PBS solution (Example 175: PBS in Fig. 4) was prepared by mixing 100 µL of the solution with 900 µL of 1 ×PBS (pH 7.4).

After 13.6 g of Plu. was weighed, the Plu. was added to 66.4 g of 1×PBS (pH 7.4), and dissolved at approximately 4°C to prepare a 17% Plu.-PBS base solution. After 14 mg of FITC-DEX was dissolved in the 17% Plu.-PBS base solution, the volume was adjusted to 10 mL to prepare a 0.14%(w/v) FITC-DEX-containing 17% Plu.-PBS solution (Example 176: 17% Plu./PBS in Fig. 4).

After 4 g of Sol. was weighed, the Sol. was added to 16 g of purified water, and dissolved at approximately 4°C to prepare a 20% Sol. base solution. Further, 20 g of Sol. was weighed and added to 80 g of PBS, and dissolved at approximately 4°C to prepare a 20% Sol.-PBS base solution. A 0.14%(w/v) FITC-DEX-containing 18% Sol. solution (Example 177: 18% Sol. in Fig. 4) and a 0.14%(w/v) FITC-DEX-containing 18% Sol.-PBS solution (Example 178: 18% Sol./PBS in Fig. 4) were prepared by mixing 100 µL of the 1.4%(w/v) FITC-DEX solution with 900 µL of the 20% Sol. base solution or 20% Sol.-PBS base solution.

### Experimental Example 8: Evaluation of retention properties in ear using fluorescent dye

For each solution prepared in Preparation Example 8, a retention test in the rat ear was carried out (N=4-6). Under isoflurane (FORANE, manufactured by Abbott) inhalation anesthesia, the tympanic membrane of a rat (Crl:CD(SD), 7 weeks old) was perforated using a Rosen probe (manufactured by DAIICHI MEDICAL). Through this perforation, 50 µL of each preparation was administered using a micropipette (Reference 2, manufactured by Eppendorf). Together with the completion of administration, inhalation anesthesia was stopped, and the rat was awakened. After 24 hours, the rat was subjected to exsanguination treatment and decapitation under anesthesia, and the middle ear cavity was extracted. FITC-DEX in the extracted middle ear cavity was collected, and the residual rate of FITC-DEX in the ear was measured using a fluorescent plate reader (Spectra Max i3x, manufactured by Molecular Devices).

The results are shown in Fig. 4. The error bar in Fig. 4 means standard deviation. The residual rate after 24 hours from administration was less than 10% in the 0.14%(w/v) FITC-DEX-containing PBS solution (Example 175), approximately 14% in the 0.14%(w/v) FITC-DEX-containing 17%Plu.-PBS solution (Example 176), and approximately 40-60% in the 18% Sol. solutions (Example 177 and Example 178).

### Experimental Example 9: Evaluation of retention properties in ear by PET imaging

In order to evaluate the retention properties in the ear of the Sol. base solution or the Sol.-trisodium citrate base solution, the retention properties in the ear was evaluated by PET imaging using ⁸⁹Zr-HB-EGF, which was obtained by labeling rat HB-EGF with ⁸⁹Zr, as a drug. Various solutions were prepared by incorporating ⁸⁹Zr-labeled HB-EGF into a Sol. base solution, a Sol.-trisodium citrate base solution, a chitosan base solution, or a Plu.-PBS base solution. Each of the solutions was administered into the external auditory canal of a mouse, and after leaving the mouse alone for a certain period of time, PET imaging was carried out. The amount of HB-EGF was quantitatively determined from the PET images thus obtained, and the retention properties in the ear was evaluated by calculating the residual rate of radioactivity in the ear from the calculated residual amount.

### <Preparation Example 9-1> Preparation of 20% Sol. base solution

After 4 g of Sol. was weighed, the Sol. was added to 16 g of purified water, and dissolved at approximately 4°C to prepare a 20% Sol. base solution.

### <Preparation Example 9-2> Preparation of 20% Sol.-2.1% trisodium citrate base solution

To prepare 20 mL of a trisodium citrate solution, 420 mg of trisodium citrate was added to purified water. After 4 g of Sol. was weighed, the Sol. was added to 16 g of the solution, and dissolved at approximately 4°C to prepare a 20% Sol.-2.1% trisodium citrate base solution.

### <Preparation Example 9-3> Preparation of 18.9% Plu.-PBS base solution

After 3.78 g of Plu. was weighed, the Plu. was added to 16.22 g of 1×PBS (pH 7.4), and dissolved at approximately 4°C to prepare an 18.9% Plu.-PBS base solution.

### <Preparation Example 9-4> Preparation of chitosan base solution

A chitosan base solution was prepared by mixing 45 µL of fibrinogen (manufactured by Enzyme Research Lab) into 455 µL of chitosan-lactic acid prepolymer (manufactured by Auration (Stanford University))(Kim et al., J Biomed Mater Res B Appl Biomater. 2014 Oct; 102(7): 1393-1406.).

### <Preparation Example 9-5> Preparation of 0.2% PS20-containing PBS solution

After 40 mg of PS20 was weighed, the PS20 was dissolved in 1×PBS (pH 7.4) to prepare 20 mL of a 0.2% PS20-containing PBS solution.

### <Preparation Example 9-6> Preparation of DFO-HB-EGF

After 500 µg of rat HB-EGF (cyt-170, manufactured by Prospec) was dissolved in 500 µL of a 0.1 mol/L sodium hydrogen carbonate solution, the solution was mixed with 10 µL of a 40 mmol/L p-SCN-Bn-Deferoxamine solution, which had been prepared by dissolving p-SCN-Bn-Deferoxamine (in-house synthesized with reference to WO2008/124467) in DMSO (DMSO is an abbreviation for dimethyl sulfoxide), and mixed at a speed of 300 rpm in an incubator (Thermomixer C, manufactured by Eppendorf) set at 37°C for 30 minutes to obtain a reaction solution. After the reaction solution was applied to a PD-10 column (17085101, manufactured by GE Healthcare), elution was carried out with a 1×PBS (pH 7.4) solution to collect eluates in 500 µL portions, and the seventh and eighth fractions were collected as the target protein, DFO-HB-EGF.

### <Preparation Example 9-7> Preparation of ⁸⁹Zr-HB-EGF-containing PBS solution and ⁸⁹Zr-HB-EGF-containing PS20-PBS solution

After 18 µL of a 1 mol/L sodium carbonate solution was added to 20 µL of [⁸⁹Zr] Zr-oxalate in a 1 mol/L oxalic acid solution (10.52-38.5 MBq), 88 µL of a 0.5 mol/L HEPES solution (HEPES means hydroxyethylpiperazine ethanesulfonic acid) was added thereto and mixed. Further, 50 µL of DFO-HB-EGF (fraction 7 or 8) prepared in Preparation Example 9-6 was added thereto, and the mixture was incubated in a block incubator (BI-516s, manufactured by ASTEC) set at 37°C for 30 to 60 minutes to perform a reaction. The reaction solution was applied to an ultrafiltration membrane (Amicon Ultra 3K, manufactured by Amicon), and ultrafiltration purification was carried out by further adding PBS to obtain a ⁸⁹Zr-HB-EGF-containing PBS solution (5.80-9.09 MBq) of interest. Further, a ⁸⁹Zr-HB-EGF-containing PS20-PBS solution was prepared by mixing the obtained solution and a 0.2% PS20-containing PBS solution at 3:1(v/v).

### <Preparation Example 9-8> ⁸⁹Zr-HB-EGF-containing 18% Sol. solution

A ⁸⁹Zr-HB-EGF-containing 18% Sol. solution (Example 179) was prepared by mixing 10 to 100 µL of the ⁸⁹Zr-HB-EGF-containing PS20-PBS solution prepared in Preparation Example 9-7 and the 20% Sol. base solution prepared in Preparation Example 9-1 at a ratio of 1:9. The solution was stored under ice cooling until immediately before administration.

### <Preparation Example 9-9> Preparation of ⁸⁹Zr-HB-EGF-containing 18% Sol.-1.9% trisodium citrate solution

A ⁸⁹Zr-HB-EGF-containing 18% Sol.-1.9% trisodium citrate solution (Example 180) was prepared by mixing 10 to 100 µL of the ⁸⁹Zr-HB-EGF-containing PS20-PBS solution prepared in Preparation Example 9-7 and the 20% Sol.-2.1% trisodium citrate base solution prepared in Preparation Example 9-2 at a ratio of 1:9. The solution was stored under ice cooling until immediately before administration.

### <Preparation Example 9-10> Preparation of ⁸⁹Zr-HB-EGF-containing 17% Plu.-PBS solution

A ⁸⁹Zr-HB-EGF-containing 17% Plu.-PBS solution (Example 181) was prepared by mixing 10 µL of the ⁸⁹Zr-HB-EGF-containing PS20-PBS solution prepared in Preparation Example 9-7 and the 18.9% Plu.-PBS base solution prepared in Preparation Example 9-3 at a ratio of 1:9. The solution was stored under ice cooling until immediately before administration.

### <Preparation Example 9-11> Preparation of ⁸⁹Zr-HB-EGF-containing chitosan solution

A ⁸⁹Zr-HB-EGF-containing chitosan solution was prepared by mixing 10 µL of the ⁸⁹Zr-HB-EGF-containing PBS solution prepared in Preparation Example 9-7 with 90 µL of the chitosan base solution prepared in Preparation Example 9-4.

### <Experimental Example 9> PET imaging

After mice (BALB/C, 9-11 weeks old) was anesthetized with isoflurane, 3 µL of each solution obtained in Preparation Examples 9-8, 9-9, and 9-10 was administered to the external auditory canal (⁸⁹Zr-HB-EGF-containing 18% Sol. solution: N=15, ⁸⁹Zr-HB-EGF-containing 18% Sol.-1.9% trisodium citrate solution: N=15, ⁸⁹Zr-HB-EGF-containing 17% Plu.-PBS solution: N=9). With respect to the ⁸⁹Zr-HB-EGF-containing chitosan solution prepared in Preparation Example 9-11, 2.55 µL thereof was administered, and 0.45 µL of 0.2 mol/L sodium metabisulfite (manufactured by Sigma-Aldrich) was further administered to polymerize the ⁸⁹Zr-HB-EGF-containing chitosan solution in the ear (Example 182)(N=9). After 0.15 to 0.31 hour, 1 day, and 2 days from the administration, images were obtained using a PET/CT scanner (PET: Clairvivo PET, manufactured by SHIMADZU CORPORATION, CT: Aquilion, manufactured by TOSHIBA CORPORATION) under anesthesia. The radioactivity residual rate after 1 day and 2 days from the administration with respect to the radioactivity in the ear immediately after administration was calculated.

The results are shown in Fig. 5. The error bar in Fig. 5 means standard error. After 2 days from the administration, the ⁸⁹Zr-HB-EGF-containing 18% Sol.-1.9% trisodium citrate solution (Example 180) showed a higher radioactivity residual rate than other three solutions. The residual rate in the ⁸⁹Zr-HB-EGF-containing chitosan solution (Example 182) decreased by approximately 50% from 1 day after administration to 2 days after administration, and the residual rate in the ⁸⁹Zr-HB-EGF-containing 17% Plu.-PBS solution (Example 181) decreased by approximately 18% from 1 day after administration to 2 days after administration. In the ⁸⁹Zr-HB-EGF-containing 18% Sol. solution (Example 179) and ⁸⁹Zr-HB-EGF-containing 18% Sol.-1.9%trisodium citrate solution (Example 180), each residual rate decreased by less than 10% from 1 day after administration to 2 days after administration.

### Experiment 10: Evaluation of efficacy of HB-EGF-containing solution using chronic tympanic membrane perforation model

A mouse model of chronic tympanic membrane perforation was prepared, and the efficacy for chronic tympanic membrane perforation was evaluated when each mouse HB-EGF-containing solution was administered.

### <Experimental Example 10-1> Preparation of mouse model of chronic tympanic membrane perforation

Under isoflurane (manufactured by Pfizer) anesthesia, the tympanic membrane of mouse (male CBA/CaJ species, 9-13 weeks old) was subjected to total perforation using a Rosen probe (52-147-50, manufactured by DAIICHI MEDICAL). GELFOAM (registered trademark) (manufactured by Pfizer) soaked with a 10 mmol/L KB-R7785 solution (in-house synthesized) (Hirayama et al., Bioorg Med Chem, 1997, Apr; 5(4) 765-778) as a perforating agent was placed through and onto the created perforation. In connection with this, the GELFOAM (registered trademark) was used after being cut into a size that could go into the ear of the mouse. The inside of the ear was observed once a day, and in a case where the GELFOAM (registered trademark) had disappeared, fresh GELFOAM (registered trademark) soaked with the perforating agent was newly placed. In a case where the GELFOAM was retained without disappearing, the 10 mmol/L KB-R7785 solution was additionally administered. The treatment with the perforating agent was carried out for one week, subsequently the perforation was left alone for approximately three months, and the perforation was checked with a microscope.

### <Preparation Example 10-1> Preparation of mouse HB-EGF solution

Mouse HB-EGF (cyt-068, manufactured by Prospec) was used as HB-EGF, and was dissolved in purified water to prepare a mouse HB-EGF solution.

### <Preparation Example 10-2> Preparation of chitosan solution (with/without mouse HB-EGF)

A chitosan base solution was prepared by mixing 45 µL of fibrinogen (manufactured by Enzyme Research Lab) into 455 µL of chitosan-lactic acid prepolymer (manufactured by Auration (Stanford University)). A chitosan solution (with mouse HB-EGF)(Example 183) or a chitosan solution (without mouse HB-EGF)(Example 184) were prepared by mixing 58.824 µg/mL of the mouse HB-EGF solution prepared in Preparation Example 10-1 or purified water with the chitosan base solution at a ratio of 1:9. These solutions were stored at 4°C.

### <Preparation Example 10-3> Preparation of 20% Sol. solution (with/without mouse HB-EGF)

After 6.7 g of Sol. was weighed, the Sol. was added to 23.3 g of purified water, and dissolved at approximately 4°C to prepare a 22.3% Sol. base solution. A 20% Sol. solution (with mouse HB-EGF)(Example 185) or a 20% Sol. solution (without mouse HB-EGF)(Example 186) was prepared by mixing 50 µg/mL of the mouse HB-EGF solution prepared in Preparation Example 10-1 or purified water with the 22.3% Sol. base solution at a ratio of 1:9.

### <Preparation Example 10-4> Preparation of 18% Sol.-1.9% trisodium citrate solution (with/without mouse HB-EGF)

After 420 mg of trisodium citrate was weighed, the trisodium citrate was dissolved in purified water to prepare 20 mL of a trisodium citrate solution. After 2 g of Sol. was weighed, the Sol. was added to 8 g of the solution, and dissolved at approximately 4°C to prepare a 20% Sol.-2.1% trisodium citrate base solution. A 18% Sol.-1.9% trisodium citrate solution (with mouse HB-EGF)(Example 187) or a 18% Sol.-1.9% trisodium citrate solution (without mouse HB-EGF)(Example 188) by mixing 50 µg/mL of the mouse HB-EGF solution prepared in Preparation Example 10-1 or purified water with the 20% Sol.-2.1% trisodium citrate base solution at a ratio of 1:9.

### <Experimental Example 10-2> Treatment of chronic tympanic membrane perforation

To the mouse model of chronic tympanic membrane perforation prepared in Experimental Example 10-1, 5 µl of each of the solutions prepared in Preparation Examples 10-2 to 10-4 (Examples 183 to 188) was administered onto the tympanic membrane using a MICROMAN E (M10E, manufactured by Gilson)(N=13-14). In connection with this, the chitosan solutions (with/without mouse HB-EGF) were respectively returned to room temperature before administration, and at the time of administering, 4.25 µL of each of the chitosan solutions and 0.75 µL of 0.2 mol/L sodium metabisulfite (manufactured by Sigma-Aldrich) were polymerized in the ear (N=13). More particularly, first, the chitosan solution (with/without mouse HB-EGF) was administered onto the tympanic membrane through the external auditory canal using a MICROMAN E, subsequently sodium metabisulfite was similarly administered, and the MICROMAN E was rapidly pulled out from the ear before the preparation hardened.

After one month from the administration, the surrounding tissue including the tympanic membrane was sampled, residue was removed, and then the presence or absence of the perforation was checked. Then, the tympanic membrane perforation healing rate was calculated from the number of ears having tympanic membrane perforation and the number of ears with completely healed tympanic membrane.

**[Table 18]**

| | Example 183 | Example 184 | Example 185 | Example 186 | Example 187 | Example 188 |
|---|---|---|---|---|---|---|
| | Chitosan solution | | 20% Sol. solution | | 18% Sol.-1.9% trisodium citrate solution | |
| Mouse HB-EGF (5 µg/mL) | Added | Not added | Added | Not added | Added | Not added |
| Number of ears having tympanic membrane perforation | 4 | 11 | 4 | 12 | 2 | 11 |
| Number of ears with completely healed tympanic membrane | 9 | 2 | 9 | 1 | 12 | 3 |
| Healing rate (%) | 69 | 15 | 69 | 8 | 86 | 21 |

The results are shown in Table 18. The healing rates of tympanic membrane perforation by the chitosan solution (containing mouse HB-EGF) (Example 183) and the 20% Sol. solution (containing mouse HB-EGF)(Example 185) were 69%. The healing rate of tympanic membrane perforation by the 18% Sol.-1.9% trisodium citrate solution (containing mouse HB-EGF)(Example 187) was 86%.

From the above results, according to the pharmaceutical composition of the present invention, since mixing is unnecessary at the time of administration and it does not gelate even at room temperature, a pharmaceutical composition that can be administered into the ear without any complicated operation and has a function of allowing a drug to be retained and slowly released in the ear can be provided.

### INDUSTRIAL APPLICABILITY

According to the present invention, there can be provided a pharmaceutical composition that can be administered into the ear without any complicated operation and has a function of allowing a drug to be retained and slowly released in the ear.

Although the present invention has been described with reference to specific embodiments, various changes and modifications obvious to those skilled in the art are possible without departing from the scope of the appended claims.

## Claims

1. A pharmaceutical composition for otic administration, comprising one, or two or more drugs and a polymer, wherein when a complex viscosity of the pharmaceutical composition is measured using a rheometer under the conditions of a shear strain of 5% and an angular frequency of 50 rad/sec., while increasing the temperature from 25°C to 37°C at a heating rate of 1°C/10 sec., the complex viscosity is less than 1,650 mPa·s at 25°C, and is from 1,650 mPa·s to 100,000 mPa·s at 10 minutes after reaching 37°C.

2. The pharmaceutical composition according to claim 1, wherein a sol-gel transition point is 30°C to 38°C.

3. The pharmaceutical composition according to claim 1 or 2, wherein the polymer is a polyvinyl caprolactam-polyvinyl acetate-polyethylene glycol graft copolymer.

4. The pharmaceutical composition according to any one of claims 1 to 3, further comprising one, or two or more agents for adjusting complex viscosity.

5. The pharmaceutical composition according to claim 4, wherein the agent for adjusting complex viscosity is one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof.

6. The pharmaceutical composition according to claim 4 or 5, wherein the agent for adjusting complex viscosity is one, or two or more compounds selected from the group consisting of trisodium citrate, tripotassium citrate, sodium sulfate, and hydrates thereof.

7. The pharmaceutical composition according to any one of claims 1 to 6, further comprising a solvent.

8. The pharmaceutical composition according to any one of claims 4 to 7, wherein a concentration of the polyvinyl caprolactam-polyvinyl acetate-polyethylene glycol graft copolymer is 10%(w/w) to 24%(w/w) with respect to the pharmaceutical composition.

9. The pharmaceutical composition according to any one of claims 4 to 8, wherein a concentration of the agent for adjusting complex viscosity is 0.1%(w/w) to 5.0%(w/w) with respect to the pharmaceutical composition.

10. The pharmaceutical composition according to any one of claims 1 to 9, wherein the drug is slowly released, when a thin, semipermeable membrane-attached insert is inserted into a multi-well plate containing 2 mL of a phosphate buffer at 37°C ± 2°C so that the phosphate buffer is divided into an upper layer and a lower layer, 0.5 mL of a pharmaceutical composition is added to the insert of upper layer, and the drug is dissolved by shaking the multi-well plate at a rate at which the water surface of the phosphate buffer shakes.

11. The pharmaceutical composition according to any one of claims 1 to 10, wherein the drug is one, or two or more compounds selected from the group consisting of a small molecule compound, a nucleic acid, and a protein.

12. The pharmaceutical composition according to any one of claims 1 to 11, wherein the drug is a drug that provides a biological activity of a heparin-binding epidermal growth factor.

13. The pharmaceutical composition according to claim 12, wherein the drug that provides a biological activity of a heparin-binding epidermal growth factor comprises a protein consisting of the amino acid sequence of SEQ ID NO: 1.

14. A pharmaceutical composition for otic administration, comprising one, or two or more drugs, and a polyvinyl caprolactam-polyvinyl acetate-polyethylene glycol graft copolymer.

15. The pharmaceutical composition according to claim 14, further comprising one, or two or more agents for adjusting complex viscosity.

16. The pharmaceutical composition according to claim 15, wherein the agent for adjusting complex viscosity is one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof.

17. The pharmaceutical composition according to claim 15 or 16, wherein the agent for adjusting complex viscosity is one, or two or more compounds selected from the group consisting of trisodium citrate, tripotassium citrate, sodium sulfate, and hydrates thereof.

18. The pharmaceutical composition according to any one of claims 14 to 17, further comprising a solvent.

19. The pharmaceutical composition according to any one of claims 15 to 18, wherein a concentration of the polyvinyl caprolactam-polyvinyl acetate-polyethylene glycol graft copolymer is 10%(w/w) to 24%(w/w) with respect to the pharmaceutical composition.

20. The pharmaceutical composition according to any one of claims 15 to 19, wherein a concentration of the agent for adjusting complex viscosity is 0. 1%(w/w) to 5.0%(w/w) with respect to the pharmaceutical composition.

21. The pharmaceutical composition according to any one of claims 14 to 20, wherein the drug is one, or two or more compounds selected from the group consisting of a small molecule compound, a nucleic acid, and a protein.

22. The pharmaceutical composition according to any one of claims 14 to 21, wherein the drug is a drug that provides a biological activity of a heparin-binding epidermal growth factor.

23. The pharmaceutical composition according to claim 22, wherein the drug that provides a biological activity of a heparin-binding epidermal growth factor comprises a protein consisting of the amino acid sequence of SEQ ID NO: 1.

24. A pharmaceutical composition for otic administration, comprising a drug that provides a biological activity of a heparin-binding epidermal growth factor, a polyvinyl caprolactam-polyvinyl acetate-polyethylene glycol graft copolymer, and trisodium citrate or a hydrate thereof.

25. Use of a polyvinyl caprolactam-polyvinyl acetate-polyethylene glycol graft copolymer, as a base, for the manufacture of a pharmaceutical composition for otic administration comprising one, or two or more drugs.

26. Use of one, or two or more compounds selected from the group consisting of trisodium citrate, tripotassium citrate, triammonium citrate, disodium succinate, sodium carbonate, sodium tartrate, sodium sulfate, ammonium sulfate, sodium dihydrogen citrate, sodium dihydrogen phosphate, disodium hydrogen citrate, potassium dihydrogen citrate, diammonium hydrogen citrate, sodium tetraborate, sodium acetate, and hydrates thereof, as an agent for adjusting complex viscosity, for the manufacture of a pharmaceutical composition for otic administration comprising one, or two or more drugs, and a polyvinyl caprolactam-polyvinyl acetate-polyethylene glycol graft copolymer.
